# EUROPEAN PATENT SPECIFICATION

(11) **EP 3 413 807 B1**
(45) Date of publication and mention of the grant of the patent: **16.08.2023**
(21) Application number: 17750658.1
(22) Date of filing: 08.02.2017
(51) Int. Cl.: A61B 17/064, A61B 17/068, A61B 17/10, A61B 17/00, A61B 90/00

(54) **FIXATION STAPLES FOR USE IN SURGICAL PROCEDURES**
FIXIERUNGSKLAMMERN ZUR VERWENDUNG BEI CHIRURGISCHEN EINGRIFFEN
AGRAFES DE FIXATION À UTILISER DANS DES PROCÉDURES CHIRURGICALES

(30) Priority: 08.02.2016 US 201662292823 P; 27.06.2016 US 201662355276 P
(43) Date of publication of application: 19.12.2018
(73) Proprietor: Acumed LLC, Hillsboro, OR 97124 (US)
(72) Inventor: KRUMME, John, Woodside California 94062 (US)
(74) Representative: Roberts, Michael Austin
(86) International application number: PCT/US2017/016914
(87) International publication number: WO 2017/139315

(56) References cited:
- EP-A1- 3 095 393
- EP-A1- 3 273 872
- WO-A1-2009/091770
- US-A- 3 960 147
- US-A- 4 411 378
- US-A1- 2009 254 090
- US-A1- 2012 024 937
- US-A1- 2012 228 355
- US-A1- 2013 030 438
- US-A1- 2014 358 187

## Description

### TECHNICAL FIELD

The present disclosure relates to staples used in surgical procedures, particularly for bone-to-bone and bone-to-tissue fixation. More specifically, the present disclosure relates to delivery devices that include a staple and an inserter, and staples that include a base member and a reinforcing member. Those of skill in the art will appreciate that the disclosed technology may be employed in contexts beyond the staples disclosed herein.

### BACKGROUND

Staples of various designs are used for fixation in surgical procedures. In such procedures, two body parts on either side of an interface are joined together by drilling holes in the body parts on either side of the interface and inserting the legs of a staple into the holes. The body parts may be bones, ligaments, tendons, or other tissues. The interface may be a natural joint, a natural epiphysis, an osteotomy, a resection, a fracture, an avulsion, or another discontinuity between the body parts. The body parts may be human or animal. The holes may be parallel, converging, or diverging. The legs of the staple may be substantially parallel to each other when they are inserted into the holes, but the staple is constructed so that after the staple has been implanted, the ends of the legs converge forcefully towards each other, and thus compress and substantially immobilize the interface. Continuing compression of the body parts has additional benefits, for example continuing compression of bones at the interface promotes bone regrowth. The known surgical staples are composed of a shape memory metal, such as a nickel titanium alloy, or an elastic polymeric material, such as polyetherether ketone (PEEK), polymeric compositions based on PEEK, polytetrafluoroethylene (such as the material sold under the trade name TEFLON^{™}), polymethylmethacrylate (PMMA), polyamides, and polyacetals.

US2012/228355 A1 discloses a surgical instrument including an implant, a grip, and a pre-set breaking point connecting the implant and the grip. US2009/254090 A1 discloses a compression staple including two arms connected by a transverse bridge. Each arm includes an anchor portion and a manipulation portion; arranged so as to extend from one another and connected by a separation mechanism or breakawayi feature. The anchor portion is used to anchor the arm in a bone while the manipulation portion is used to guide the arm in displacement relative to the bridge.

### SUMMARY

The invention is defined by the appended claims.

The various systems and methods of the present technology have been developed in response to the present state of the art, and in particular, in response to the problems and needs in the art that have not yet been fully solved by currently available staples and inserters.

In an aspect of the technology, a staple delivery device includes: a staple including a bridge, a first leg, and a second leg, wherein the bridge extends between a first end and a second end, wherein the first leg extends transversely from the first end of the bridge and terminates in a first free end which is remote from the bridge, wherein the second leg extends transversely from the second end of the bridge and terminates in a second free end which is remote from the bridge, wherein the first free end is beside the second free end; and an inserter connected to the staple, wherein the inserter includes a first body and a second body which is a mirror image of the first body, wherein the first body is connected to the first end of the bridge by a first connection, wherein the second body is connected to the second end of the bridge by a second connection, wherein the first and second bodies are joined together at a junction which is adjacent to the bridge opposite the first and second legs; wherein the inserter is disconnected from the staple by rupturing the first and second connections, wherein the first and second connections are ruptured by an action selected from the group consisting of cutting the first and second connections with a surgical wire cutter, cutting the first and second connections with scissors, cutting the first and second connections with a scalpel, breaking the first and second connections with a rupturing instrument, and breaking the first and second connections manually.

In another aspect of the technology, a staple delivery device includes: a staple including a bridge, a first leg, and a second leg, wherein the bridge extends between a first end and a second end, wherein the first leg extends transversely from the first end of the bridge and terminates in a first free end which is remote from the bridge, wherein the second leg extends transversely from the second end of the bridge and terminates in a second free end which is remote from the bridge, wherein the first free end is beside the second free end; and an inserter integrally formed as a single part with the staple, wherein the inserter includes a first body and a second body which is a mirror image of the first body, wherein the first body is connected to the first end of the bridge by a first connection, wherein the second body is connected to the second end of the bridge by a second connection, wherein the first and second bodies are joined together at a junction which is adjacent to the bridge opposite the first and second legs; wherein the inserter is disconnected from the staple by rupturing the first and second connections, wherein the first and second connections are ruptured by an action selected from the group consisting of cutting the first and second connections with a surgical wire cutter, cutting the first and second connections with scissors, cutting the first and second connections with a scalpel, breaking the first and second connections with a rupturing instrument, and breaking the first and second connections manually.

In yet another aspect of the technology, a staple delivery device includes: a staple including a bridge, a first leg, a second leg, a first securing member, and a second securing member, wherein the bridge extends between a first end and a second end, wherein the first leg extends transversely from the first end of the bridge and terminates in a first free end which is remote from the bridge, wherein the second leg extends transversely from the second end of the bridge and terminates in a second free end which is remote from the bridge, wherein the first free end is beside the second free end, wherein the first securing member extends transversely from the first end of the bridge opposite the first leg, wherein the second securing member extends transversely from the second end of the bridge opposite the second leg; and an inserter connected to the staple, wherein the inserter includes a first body and a second body which is a mirror image of the first body, wherein the first body includes a first recess, wherein the second body includes a second recess, wherein the first recess receives the first securing member to form a first connection, wherein the second recess receives the second securing member to form a second connection, wherein the first and second bodies are joined together at a junction which is adjacent to the bridge opposite the first and second legs; wherein the inserter is disconnected from the staple by rupturing the first and second connections, wherein the first and second connections are ruptured by an action selected from the group consisting of cutting the first and second connections with a surgical wire cutter, cutting the first and second connections with scissors, cutting the first and second connections with a scalpel, breaking the first and second connections with a rupturing instrument, and breaking the first and second connections manually.

These and other features and advantages of the present technology will become more fully apparent from the following description and appended claims, or may be learned by the practice of the technology as set forth hereinafter.

### BRIEF DESCRIPTION OF THE DRAWINGS

Exemplary embodiments of the technology will become more fully apparent from the following description and appended claims, taken in conjunction with the accompanying drawings. Understanding that these drawings depict only exemplary embodiments and are, therefore, not to be considered limiting of the scope of the technology, the exemplary embodiments will be described with additional specificity and detail through use of the accompanying drawings in which:
FIG. 1 is a front view of a delivery device;
FIG. 2 is a side view of the delivery device of FIG. 1;
FIG. 3 is a front view of another delivery device;
FIG. 4 is a side view of the delivery device of FIG. 3;
FIG. 5 is a perspective view of yet another delivery device;
FIG. 6A is a perspective view of yet another delivery device; FIG. 6B is another perspective view of the delivery device of FIG. 6A from a different direction; FIG. 6C is an exploded perspective view of the delivery device of FIG. 6A; and FIG. 6D is another exploded perspective view of the delivery device of FIG. 6A from a different direction;
FIG. 7 is a perspective view of yet another delivery device;
FIG. 8 is a front view of yet another delivery device;
FIG. 9 is a front view of a staple;
FIG. 10 is a perspective view of the staple of FIG. 9;
FIG. 11 is a perspective view of another staple;
FIG. 12 is an exploded perspective view of the staple of FIG. 11;
FIG. 13A is a perspective view of yet another staple, not forming part of the claimed invention; FIG. 13B is a front view of the staple of FIG. 13A; and FIG. 13C is a cross sectional view of a base member of the staple of FIG. 13A taken along section line 13C-13C of FIG. 13B;
FIG. 14A is a perspective view of yet another staple, not forming part of the claimed invention; FIG. 14B is a front view of the staple of FIG. 14A; and FIG. 14C is a cross sectional view of the staple of FIG. 14A taken along section line 14C-14C of FIG. 14B;
FIG. 15 is a perspective view of yet another staple, not forming part of the claimed invention; and
FIG. 16 is a perspective view of yet another staple, not forming part of the claimed invention;

### DETAILED DESCRIPTION

Exemplary embodiments of the technology will be best understood by reference to the drawings, wherein like parts are designated by like numerals throughout. It will be readily understood that the components of the technology, as generally described and illustrated in the figures herein, could be arranged and designed in a wide variety of different configurations. Thus, the following more detailed description of the embodiments of the apparatus, system, and method is not intended to limit the scope of the invention, as claimed, but is merely representative of exemplary embodiments of the technology.

The phrases "connected to," "coupled to" and "in communication with" refer to any form of interaction between two or more entities, including mechanical, electrical, magnetic, electromagnetic, fluid, and thermal interaction. Two components may be functionally coupled to each other even though they are not in direct contact with each other. The term "abutting" refers to items that are in direct physical contact with each other, although the items may not necessarily be attached together. The phrase "fluid communication" refers to two features that are connected such that a fluid within one feature is able to pass into the other feature.

The word "exemplary" is used herein to mean "serving as an example, instance, or illustration." Any embodiment described herein as "exemplary" is not necessarily to be construed as preferred or advantageous over other embodiments. While the various aspects of the embodiments are presented in drawings, the drawings are not necessarily drawn to scale unless specifically indicated.

Standard medical planes of reference and descriptive terminology are employed in this specification. A sagittal plane divides a body into right and left portions. A mid-sagittal plane divides the body into bilaterally symmetric right and left halves. A coronal plane divides a body into anterior and posterior portions. A transverse plane divides a body into superior and inferior portions. The sagittal, coronal, and transverse planes are mutually perpendicular. Anterior means toward the front of the body. Posterior means toward the back of the body. Superior means toward the head. Inferior means toward the feet. Medial means toward the midline of the body. Lateral means away from the midline of the body. Axial means toward a central axis of the body. Abaxial means away from a central axis of the body. Ipsilateral means on the same side of the body. Contralateral means on the opposite side of the body. These descriptive terms may be applied to an animate or inanimate body.

In this specification, "elastic" is defined as behavior of a material or a part that returns to its undeformed state when applied forces are removed. "Elastic zone" is defined as a region of a stress-strain curve for a material in which the material returns to its undeformed state when applied forces are removed. The elastic zone is between zero stress, zero strain and the yield point of the material.

The numbers (dimensions) in this specification are to be construed with the latitude appropriate to their context and expression. Each number (dimension) is subject to variation which depends on the accuracy with which it can be measured by methods conventionally used by those skilled in the art as of the filing date of this disclosure. "About," "substantially," and other such terms in this specification are to be construed according to these principles.

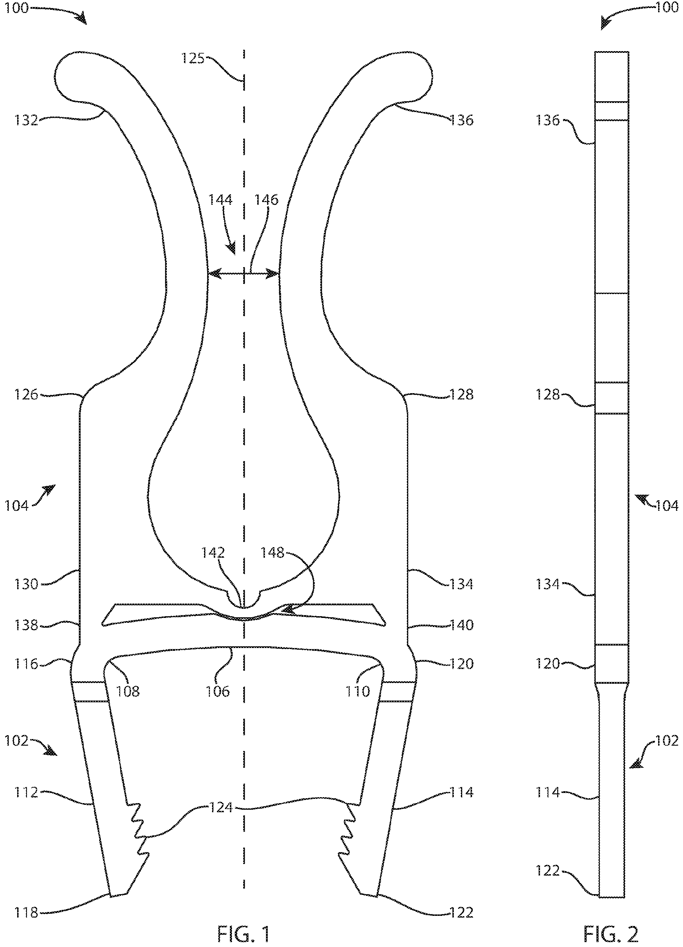

Referring to FIGS. 1 and 2, a delivery device 100 includes a staple 102 and an inserter 104. In this example, the staple 102 and inserter 104 are integrally formed as a monolithic delivery device 100. The staple 102 and inserter 104 may be made of a polymer such as PEEK or a polymeric composition based upon PEEK. Alternatively, the staple 102 may be made of a first polymer and the inserter 104 may be made of a second polymer which is different from the first polymer. In this arrangement, the staple 102 and the inserter 104 may be coupled together to form the delivery device 100, for example by bonding, for example by melt bonding. The staple 102 and inserter 104 may also be made of metal, such as a nickel titanium alloy.

The staple 102 includes a bridge 106 which extends longitudinally between a left end 108 and a right end 110. The staple 102 includes a left leg 112 and a right leg 114. A proximal end 116 of the left leg 112 is attached to the left end 108 of the bridge 106. The left leg 112 terminates in a distal end 118, which is a free end opposite the bridge 106. A proximal end 120 of the right leg 114 is attached to the right end 110 of the bridge 106. The right leg 114 terminates in a distal end 122, which is a free end opposite the bridge 106. The facing surfaces of the legs 112, 114 include teeth 124. When the staple 102 is implanted, the teeth 124 help to retain the staple 102 in the desired position. The teeth 124 may be on any or all sides of the legs 112, 114.

The staple 102 is shown in its free state, in which the staple 102 is not elastically or plastically deflected or deformed. The free state may be referred to as the closed state. In one example of the free state, the staple 102 experiences no external loads, other than gravity perhaps. However, in the free state, the staple 102 may experience loads that are below a threshold for elastic or plastic deflection or deformation. In the free state, the distal ends 118, 122 of the legs 112, 114 are closer together than the proximal ends 116, 120 so that the legs 112, 114 converge as they extend away from the bridge 106.

The staple 102 may be moved between the free state and an elastically deformed state by elastically deforming some or all of the staple 102. For example, elastically deflecting or pressing on the bridge 106 so that it is straight or curves down between the legs 112, 114 causes the distal ends 118, 122 to spread apart. The legs 112, 114 may spread apart in the elastically deformed state so as to become parallel. The elastically deformed state may be referred to as the open state. The elastically deformed state may be suitable for implantation of the staple 102 and may thus be referred to as an insertion state or an implantation state.

The inserter 104 may be referred to as a delivery member. The inserter 104 includes a left body 126 and a right body 128 which in this example is a mirror image of the left body 126 across a first plane of symmetry 125, which is shown edge on in FIG. 1 and is thus represented by a dashed line 125. The left body 126 includes a distal portion 130 and a proximal portion 132. The right body 128 includes a distal portion 134 and a proximal portion 136. The left distal portion 130 is connected to the left end 108 of the bridge 106 by a left connection 138. The right distal portion 134 is connected to the right end 110 of the bridge 106 by a right connection 140. The distal portions 130, 134 are connected to the bridge 106 solely by the connections 138, 140, which are at or close to the ends 108, 110. The connections 138, 140 may be ruptured after the staple 102 has been implanted, to disconnect the inserter 104 from the staple 102. For example, the connections 138, 140 may be ruptured by use of a surgical wire cutter, scissors, a scalpel, or a rupturing instrument specifically designed to correspond to the connections 138, 140, or by being manually broken. After rupture, the bridge 106 may include residue from the ruptured connections 138, 140. The distal portions 130, 134 extend along and above the bridge 106 and are joined together at a central junction 142 which is shown in the vicinity of the plane 125. The central junction 142 is not secured to the bridge 106, but can be brought into contact with the bridge 106 as discussed below.

The inserter 104 is shown in its free state, in which the inserter 104 is not elastically or plastically deflected or deformed. In one example of the free state, the inserter 104 experiences no external loads, other than gravity perhaps. However, in the free state, the inserter 104 may experience loads that are below a threshold for elastic or plastic deflection or deformation. In the free state, the proximal portions 132, 136 are separated by a gap 144 having a free state dimension 146. In the free state, the central junction 142 may contact the bridge 106, or the central junction 142 may optionally be separated from the bridge by a gap 148.

The inserter 104 may be moved between the free state and an actuated state by moving or pressing the proximal portions 132, 136 towards each other and towards the plane 125, thereby changing the shape of the inserter 104. This can be done manually, for example with an index finger on the left proximal portion 132 and a thumb on the right proximal portion 136. In other examples, the proximal portions 132, 136 may be moved away from each other or otherwise moved relative to each other, to move the inserter 104 between the free state and the actuated state. The actuated state may be referred to as a compressed state, an insertion state, or an implantation state. When the inserter 104 is moved from the free state to the actuated state, the gap 144 becomes smaller, the bodies 126, 128 pivot about the connections 138, 140 respectively, and the central junction 142 presses against the bridge 106, thus causing the staple 102 to move from the free state to the elastically deformed state. Conversely, as the inserter 104 is moved from the actuated state to the free state, the staple 102 moves from the elastically deformed state to the free state.

When the inserter 104 is in the actuated state and the staple 102 is in the elastically deformed state, the delivery device 100 may be used to implant the staple 102. After the staple 102 has been implanted, the connections 138, 140 may be ruptured. The inserter 104 may then be removed from the implantation site, leaving the staple 102 in place.

The height of the delivery device 100 may be 40-80 mm. The height of the staple 102 may be 18-25 mm, preferably about 21 mm. The height of the bridge 106 may be 2.5-4 mm, preferably about 3 mm. The height of the inserter 104 may be 25-40 mm, preferably about 30 mm. These heights are taken parallel to line 125 in FIG. 1. The width of the delivery device 100 may be 18-30 mm. The width of the bridge 106 may be 20-30 mm, preferably about 25 mm. These widths are taken perpendicular to line 125 in FIG. 1. The width of the bridge 106 may also be referred to as the length of the bridge 106. The free state dimension 146 of the gap 144 may be 15-30 mm, preferably about 20 mm. The dimension 146 is taken perpendicular to line 125 in FIG. 1. The thickness of the delivery device 100 may be 3-8 mm. The inserter 104, the bridge 106, and the proximal ends 116, 120 of the legs 112, 114 may be 4-7 mm thick, preferably about 5 mm thick. The distal ends 118, 122 of the legs 112, 114 may be thinner, for example 2-4 mm thick, preferably about 2.75 mm thick. These thicknesses are taken when the delivery device 100 is viewed from the side as shown in FIG. 2. Thickness may also be referred to as depth. The bridge 106, the legs 112, 114, and the inserter 104 may lie between two parallel planes.

Methods of manufacturing the delivery device 100 may include: (1) forming the staple 102 and the inserter 104 as a monolithic article by shaping a single material in a single step; (2) forming the staple 102 from a first material; forming the inserter 104 from a second material that may be the same as or different from the first material; and securing the staple 102 and the inserter 104 together; (3) forming the staple 102 from the first material; and forming the inserter 104 from the second material in contact with the previously formed staple 102; or (4) forming the inserter 104 from the second material; and forming the staple 102 from the first material in contact with the previously formed inserter 104. Shaping may involve molding, cutting, additive manufacturing processes, or the like. Molding may involve injection molding, insert molding, over molding, or the like. Cutting may involve laser cutting. Additive manufacturing may involve three-dimensional printing.

A method of using the delivery device 100, not forming part of the claimed invention, may include any or all of the following steps in any order: making a first hole in a first body part; making a second hole in a second body part; moving the inserter 104 from the free state to the actuated state; moving the staple 102 from the free state to the elastically deformed state; moving the legs 112, 114 to be parallel; inserting the left leg 112 into the first hole; inserting the right leg 114 into the second hole; seating the bridge 106 against the first and/or second body part; moving the inserter 104 from the actuated state toward the free state; moving the staple 102 from the elastically deformed state toward the free state; moving the legs 112, 114 toward a converging state; rupturing the connections 138, 140; and removing the inserter 104 from the implantation site, leaving the staple 102 in place.

Making the first and/or second holes may involve drilling the first and/or second holes, optionally using a drill guide. The first hole may be substantially parallel to the second hole. The first and/or second body parts may be bone. Moving the inserter 104 from the free state to the actuated state may occur substantially in advance of a surgical procedure in which the delivery device 100 is used or immediately before inserting the legs 112, 114 into the first and second holes, respectively. Moving the inserter 104 from the free state to the actuated state may cause the staple 102 to move from the free state to the elastically deformed state. When the staple 102 is in the elastically deformed state, the left leg 112 may be substantially parallel to the right leg 114. Inserting the legs 112, 114 into the first and second holes, respectively, may involve partially or completely provisionally inserting the legs 112, 114 into the first and second holes and partially or completely withdrawing the legs 112, 114 from the first and second holes, while the inserter 104 is in the actuated state, and/or while the staple 102 is in the elastically deformed state, and/or before rupturing the connections 138, 140. Seating the bridge 106 against the first and/or second body part may involve direct contact between a bone facing side of the bridge 106 (a surface of the bridge 106 that faces towards the distal ends 118, 122) and a surface of the first and/or second body part. Moving the inserter 104 from the actuated state toward the free state may involve releasing pressure on the proximal portions 132, 136 acting to urge the proximal portions 132, 136 towards each other and towards the plane 125. Moving the inserter 104 from the actuated state toward the free state may cause the staple 102 to move from the elastically deformed state toward the free state. The inserter 104 and the staple 102 may relax toward their respective free states, but under certain circumstances they may not completely achieve their free states. Rupturing the connections may occur while the inserter 104 is in the actuated state and/or while the staple 102 is in the elastically deformed state. Rupturing the connections may cause the staple 102 to move from the elastically deformed state toward the free state.

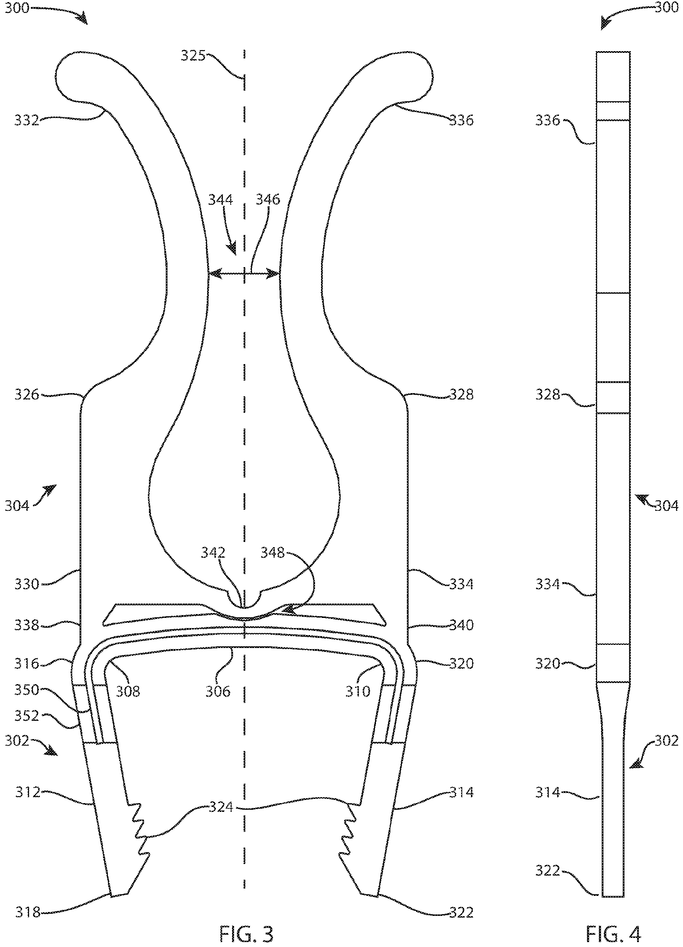

Referring to FIGS. 3 and 4, another delivery device 300 includes a staple 302 and an inserter 304. Delivery device 300 may be similar to delivery device 100. In this example, the staple 302 and inserter 304 are integrally formed as a monolithic delivery device 300. The staple 302 and inserter 304 may be made of a polymer such as PEEK or a polymeric composition based upon PEEK. Alternatively, the staple 302 may be made of a first polymer and the inserter 304 may be made of a second polymer which is different from the first polymer. In this arrangement, the staple 302 and the inserter 304 may be coupled together to form the delivery device 300, for example by bonding, for example by melt bonding. The staple 302 and the inserter 304 may also be made of metal.

The staple 302 includes a bridge 306 which extends longitudinally between a left end 308 and a right end 310. The staple 302 includes a left leg 312 and a right leg 314. A proximal end 316 of the left leg 312 is attached to the left end 308 of the bridge 306. The left leg 312 terminates in a distal end 318, which is a free end opposite the bridge 306. A proximal end 320 of the right leg 314 is attached to the right end 310 of the bridge 306. The right leg 314 terminates in a distal end 322, which is a free end opposite the bridge 306. The facing surfaces of the legs 312, 314 include teeth 324. When the staple 302 is implanted, the teeth 324 help to retain the staple 302 in the desired position. The teeth 324 may be on any or all sides of the legs 312, 314.

The staple 302 includes a reinforcing member 350 which may be metal, for example a nickel titanium alloy. The reinforcing member 350 may have a rectangular, square, or round cross section. The reinforcing member 350 may be easily fabricated from ribbon stock, square wire stock, or round wire stock. The reinforcing member 350 extends along the bridge 306 and optionally part way along the legs 312, 314. The reinforcing member 350 may extend along 50-90% of the length of each leg 312, 314. The reinforcing member 350 may be partially or wholly embedded in the bridge 306 and the legs 312, 314 of the staple 302, or it may be attached to the surface of the bridge 306 and the legs 312, 314. The staple 302 may include a base member 352, which in this example is the polymer portion of the staple 302 other than the reinforcing member 350. The base member 352 may be easily molded with the desired three-dimensional characteristics for the staple 302. The base member 352 may include a channel into which the reinforcing member 350 is fitted. The staple 302 may be referred to as a hybrid staple 302 because it includes a polymer base member 352 and a metal reinforcing member 350.

The staple 302 is shown in its free state, in which the staple 302 is not elastically or plastically deflected or deformed. The free state may be referred to as the closed state. In one example of the free state, the staple 302 experiences no external loads, other than gravity perhaps. However, in the free state, the staple 302 may experience loads that are below a threshold for elastic or plastic deflection or deformation. In the free state, the distal ends 318, 322 of the legs 312, 314 are closer together than the proximal ends 316, 320 so that the legs 312, 314 converge as they extend away from the bridge 306. The reinforcing member 350 may maintain the legs 312, 314 in the free state.

The staple 302 may be moved between the free state and an elastically deformed state by elastically deforming some or all of the staple 302, including corresponding portions of the reinforcing member 350. For example, elastically deflecting or pressing on the bridge 306 so that it is straight or curves down between the legs 312, 314 causes the distal ends 318, 322 to spread apart. The legs 312, 314 may spread apart in the elastically deformed state so as to become parallel. The elastically deformed state may be referred to as the open state. The elastically deformed state may be suitable for implantation of the staple 302 and may thus be referred to as an insertion state or an implantation state.

The inserter 304 may be referred to as a delivery member. The inserter 304 includes a left body 326 and a right body 328 which in this example is a mirror image of the left body 326 across a first plane of symmetry 325, which is shown edge on in FIG. 3 and is thus represented by a dashed line 325. The left body 326 includes a distal portion 330 and a proximal portion 332. The right body 328 includes a distal portion 334 and a proximal portion 336. The left distal portion 330 is connected to the left end 308 of the bridge 306 by a left connection 338. The right distal portion 334 is connected to the right end 310 of the bridge 306 by a right connection 340. The distal portions 330, 334 are connected to the bridge 306 solely by the connections 338, 340, which are at or close to the ends 308, 310. The connections 338, 340 may be ruptured after the staple 302 has been implanted, to disconnect the inserter 304 from the staple 302. For example, the connections 338, 340 may be ruptured by use of a surgical wire cutter, scissors, a scalpel, or a rupturing instrument specifically designed to correspond to the connections 338, 340, or by being manually broken. After rupture, the bridge 306 may include residue from the ruptured connections 338, 340. The distal portions 330, 334 extend along and above the bridge 306 and are joined together at a central junction 342 which is shown in the vicinity of the plane 325. The central junction 342 is not secured to the bridge 306, but can be brought into contact with the bridge 306 as discussed below.

The inserter 304 is shown in its free state, in which the inserter 304 is not elastically or plastically deflected or deformed. In one example of the free state, the inserter 304 experiences no external loads, other than gravity perhaps. However, in the free state, the inserter 304 may experience loads that are below a threshold for elastic or plastic deflection or deformation. In the free state, the proximal portions 332, 336 are separated by a gap 344 having a free state dimension 346. In the free state, the central junction 342 may contact the bridge 306, or the central junction 342 may optionally be separated from the bridge by a gap 348.

The inserter 304 may be moved between the free state and an actuated state by moving or pressing the proximal portions 332, 336 towards each other and towards the plane 325, thereby changing the shape of the inserter 304. This can be done manually, for example with an index finger on the left proximal portion 332 and a thumb on the right proximal portion 336. In other examples, the proximal portions 332, 336 may be moved away from each other or otherwise moved relative to each other, to move the inserter 304 between the free state and the actuated state. The actuated state may be referred to as a compressed state, an insertion state, or an implantation state. When the inserter 304 is moved from the free state to the actuated state, the gap 344 becomes smaller, the bodies 326, 328 pivot about the connections 338, 340 respectively, and the central junction 342 presses against the bridge 306, thus causing the staple 302 to move from the free state to the elastically deformed state. Conversely, as the inserter 304 is moved from the actuated state to the free state, the staple 302 moves from the elastically deformed state to the free state. The reinforcing member 350 may urge the base member 352 toward the free state and may increase the post-implantation compressive force of the staple 302 compared to unreinforced polymer staples.

When the inserter 304 is in the actuated state and the staple 302 is in the elastically deformed state, the delivery device 300 may be used to implant the staple 302. After the staple 302 has been implanted, the connections 338, 340 may be ruptured. The inserter 304 may then be removed from the implantation site, leaving the staple 302 in place.

Methods of manufacturing the delivery device 300 may include: (1) forming the base member 352 and the inserter 304 as a monolithic article by shaping a single material in a single step; forming the reinforcing member 350 of metal, preferably a nickel titanium alloy; and connecting the reinforcing member 350 to the base member 352; (2) forming the base member 352 from a first material; forming the inserter 304 from a second material that may be the same as or different from the first material; forming the reinforcing member 350 from a third material that is metal, preferably a nickel titanium alloy; and securing the base member 352, the inserter 304, and the reinforcing member 350 together; (3) forming the reinforcing member 350 from the third material; forming the base member 352 from the first material in contact with the previously formed reinforcing member 350 to form the staple 302; and forming the inserter 304 from the second material in contact with the previously formed staple 302; or (4) forming the inserter 304 from the second material; forming the reinforcing member 350 from the third material; and forming the base member 352 from the first material in contact with the previously formed inserter 304 and reinforcing member 350. The steps of forming the base member 352 and the inserter 304 as a monolithic article and connecting the reinforcing member 350 may occur simultaneously, for example as an overmolding or insert molding operation. Shaping may involve molding, cutting, additive manufacturing processes, or the like. Molding may involve injection molding, insert molding, over molding, or the like. Cutting may involve laser cutting. Additive manufacturing may involve three-dimensional printing.

A method of using the delivery device 300, not forming part of the claimed invention, may include any or all of the following steps in any order: making a first hole in a first body part; making a second hole in a second body part; moving the inserter 304 from the free state to the actuated state; moving the staple 302 from the free state to the elastically deformed state; moving the legs 312, 314 to be parallel; inserting the left leg 312 into the first hole; inserting the right leg 314 into the second hole; seating the bridge 306 against the first and/or second body part; moving the inserter 304 from the actuated state toward the free state; moving the staple 302 from the elastically deformed state toward the free state; moving the legs 312, 314 toward a converging state; rupturing the connections 338, 340; and removing the inserter 304 from the implantation site, leaving the staple 302 in place.

Making the first and/or second holes may involve drilling the first and/or second holes, optionally using a drill guide. The first hole may be substantially parallel to the second hole. The first and/or second body parts may be bone. Moving the inserter 304 from the free state to the actuated state may occur substantially in advance of a surgical procedure in which the delivery device 300 is used or immediately before inserting the legs 312, 314 into the first and second holes, respectively. Moving the inserter 304 from the free state to the actuated state may cause the staple 302 to move from the free state to the elastically deformed state. When the staple 302 is in the elastically deformed state, the left leg 312 may be substantially parallel to the right leg 314. Inserting the legs 312, 314 into the first and second holes, respectively, may involve partially or completely provisionally inserting the legs 312, 314 into the first and second holes and partially or completely withdrawing the legs 312, 314 from the first and second holes, while the inserter 304 is in the actuated state, and/or while the staple 302 is in the elastically deformed state, and/or before rupturing the connections 338, 340. Seating the bridge 306 against the first and/or second body part may involve direct contact between a bone facing side of the bridge 306 (a surface of the bridge 306 that faces towards the distal ends 318, 322) and a surface of the first and/or second body part. Moving the inserter 304 from the actuated state toward the free state may involve releasing pressure on the proximal portions 332, 336 acting to urge the proximal portions 332, 336 towards each other and towards the plane 325. Moving the inserter 304 from the actuated state toward the free state may cause the staple 302 to move from the elastically deformed state toward the free state. The inserter 304 and the staple 302 may relax toward their respective free states, but under certain circumstances they may not completely achieve their free states. Rupturing the connections may occur while the inserter 304 is in the actuated state and/or while the staple 302 is in the elastically deformed state. Rupturing the connections may cause the staple 302 to move from the elastically deformed state toward the free state.

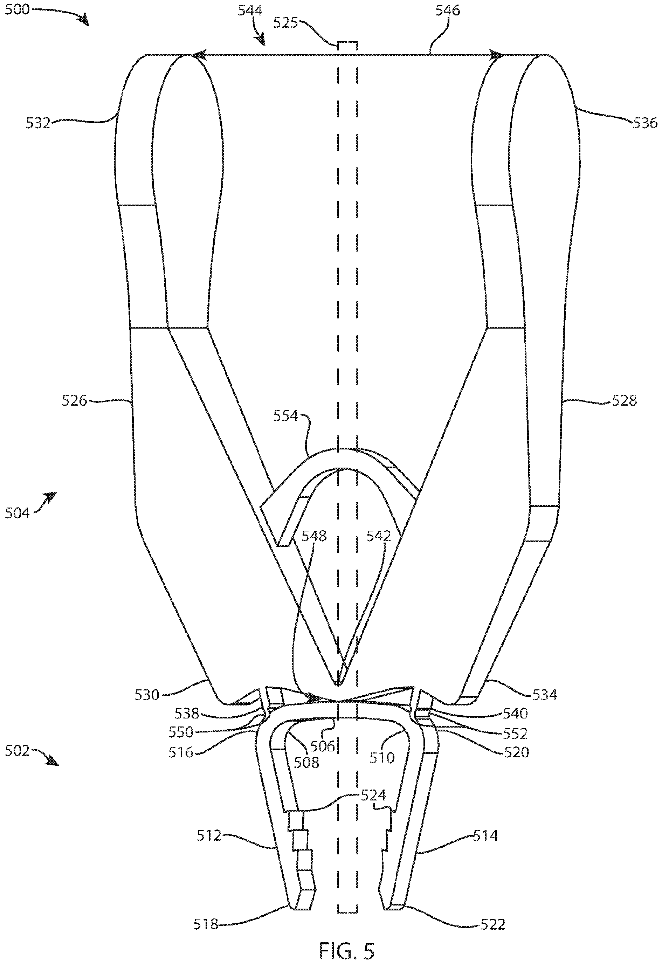

Referring to FIG. 5, yet another delivery device 500 includes a staple 502 and an inserter 504. In this example, the staple 502 and inserter 504 are integrally formed as a monolithic delivery device 500. The staple 502 and inserter 504 may be made of a polymer such as PEEK or a polymeric composition based upon PEEK. Alternatively, the staple 502 may be made of a first polymer and the inserter 504 may be made of a second polymer which is different from the first polymer. In this arrangement, the staple 502 and the inserter 504 may be coupled together to form the delivery device 500, for example by bonding, for example by melt bonding. The staple 502 and inserter 504 may also be made of metal, such as a nickel titanium alloy.

The staple 502 includes a bridge 506 which extends longitudinally between a left end 508 and a right end 510. The staple 502 includes a left leg 512 and a right leg 514. A proximal end 516 of the left leg 512 is attached to the left end 508 of the bridge 506. The left leg 512 terminates in a distal end 518, which is a free end opposite the bridge 506. A proximal end 520 of the right leg 514 is attached to the right end 510 of the bridge 506. The right leg 514 terminates in a distal end 522, which is a free end opposite the bridge 506. The facing surfaces of the legs 512, 514 include teeth 524. When the staple 502 is implanted, the teeth 524 help to retain the staple 502 in the desired position. The teeth 524 may be on any or all sides of the legs 512, 514.

The staple 502 is shown in its free state, in which the staple 502 is not elastically or plastically deflected or deformed. The free state may be referred to as the closed state. In one example of the free state, the staple 502 experiences no external loads, other than gravity perhaps. However, in the free state, the staple 502 may experience loads that are below a threshold for elastic or plastic deflection or deformation. In the free state, the distal ends 518, 522 of the legs 512, 514 are closer together than the proximal ends 516, 520 so that the legs 512, 514 converge as they extend away from the bridge 506.

The staple 502 may be moved between the free state and an elastically deformed state by elastically deforming some or all of the staple 502. For example, elastically deflecting or pressing on the bridge 506 so that it is straight or curves down between the legs 512, 514 causes the distal ends 518, 522 to spread apart. The legs 512, 514 may spread apart in the elastically deformed state so as to become parallel. The elastically deformed state may be referred to as the open state. The elastically deformed state may be suitable for implantation of the staple 502 and may thus be referred to as an insertion state or an implantation state.

The staple 502 may be modified to include a reinforcing member as described for staple 302. In this situation, the staple 502 may be referred to as a hybrid staple 502 because it includes a polymer base member and a metal reinforcing member.

The inserter 504 may be referred to as a delivery member. The inserter 504 includes a left body 526 and a right body 528 which in this example is a mirror image of the left body 526 across a first plane of symmetry 525. The left body 526 includes a distal portion 530 and a proximal portion 532. The right body 528 includes a distal portion 534 and a proximal portion 536. The left distal portion 530 is connected to the left end 508 of the bridge 506 by a left connection 538 which is noticeably thinner than the connections 138, 338. The left connection 538 includes a notch 550 at the connection with the left end 508 of the bridge 506. The notch 550 may be present on one or both sides of the left connection 538 to direct and control rupture of the left connection 538. The example shows notches 550 on both sides of the left connection 538. The right distal portion 534 is connected to the right end 510 of the bridge 506 by a thin elongated right connection 540 that includes notches 552 on both sides at the connection with the right end 510 of the bridge 506. The distal portions 530, 534 are connected to the bridge 506 solely by the connections 538, 540, which are at or close to the ends 508, 510. The connections 538, 540 may be ruptured after the staple 502 has been implanted, to disconnect the inserter 504 from the staple 502. For example, the connections 538, 540 may be ruptured at the notches 550, 552 by use of a surgical wire cutter, scissors, a scalpel, or a rupturing instrument specifically designed to correspond to the connections 538, 540, or by being manually broken. After rupture, the bridge 506 may include residue from the ruptured connections 538, 540. Preferably, the notches 550, 552 are positioned so that little or no residue protrudes from the bridge. The distal portions 530, 534 extend along and above the bridge 506 and are joined together at a central junction 542 which is shown in the vicinity of the plane 525. The central junction 542 is not secured to the bridge 506, but can be brought into contact with the bridge 506 as discussed below.

The inserter 504 includes a flex bridge 554 which extends between the bodies 526, 528. The flex bridge 554 is curved or bent so that the flex bridge 554 is deformable, preferably elastically deformable. The flex bridge 554 maintains the bodies 526, 528 in a desired spatial configuration. The flex bridge 554 may be integrally formed with the inserter 504, or it may be a separate part that is attached between the bodies 526, 528.

The inserter 504 is shown in its free state, in which the inserter 504 is not elastically or plastically deflected or deformed. In one example of the free state, the inserter 504 experiences no external loads, other than gravity perhaps. However, in the free state, the inserter 504 may experience loads that are below a threshold for elastic or plastic deflection or deformation. In the free state, the proximal portions 532, 536 are separated by a gap 544 having a free state dimension 546. In the free state, the central junction 542 may contact the bridge 506, or the central junction 542 may optionally be separated from the bridge by a gap 548.

The inserter 504 may be moved between the free state and an actuated state by moving or pressing the proximal portions 532, 536 towards each other and towards the plane 525, thereby changing the shape of the inserter 504. This can be done manually, for example with an index finger on the left proximal portion 532 and a thumb on the right proximal portion 536. In other examples, the proximal portions 532, 536 may be moved away from each other or otherwise moved relative to each other, to move the inserter 504 between the free state and the actuated state. The actuated state may be referred to as a compressed state, an insertion state, or an implantation state. When the inserter 504 is moved from the free state to the actuated state, the gap 544 becomes smaller, the bodies 526, 528 pivot about the connections 538, 540 respectively, the flex bridge 554 deforms, and the central junction 542 presses against the bridge 506, thus causing the staple 502 to move from the free state to the elastically deformed state. Conversely, as the inserter 504 is moved from the actuated state to the free state, the staple 502 moves from the elastically deformed state to the free state.

When the inserter 504 is in the actuated state and the staple 502 is in the elastically deformed state, the delivery device 500 may be used to implant the staple 502. After the staple 502 has been implanted, the connections 538, 540 may be ruptured. The inserter 504 may then be removed from the implantation site, leaving the staple 502 in place.

Methods of manufacturing the delivery device 500 may include: (1) forming the staple 502 and the inserter 504 as a monolithic article by shaping a single material in a single step; (2) forming the staple 502 from a first material; forming the inserter 504 from a second material that may be the same as or different from the first material; and securing the staple 502 and the inserter 504 together; (3) forming the staple 502 from the first material; and forming the inserter 504 from the second material in contact with the previously formed staple 502; or (4) forming the inserter 504 from the second material; and forming the staple 502 from the first material in contact with the previously formed inserter 504. Shaping may involve molding, cutting, additive manufacturing processes, or the like. Molding may involve injection molding, insert molding, over molding, or the like. Cutting may involve laser cutting. Additive manufacturing may involve three-dimensional printing.

A method of using the delivery device 500, not forming part of the claimed invention, may include any or all of the following steps in any order: making a first hole in a first body part; making a second hole in a second body part; moving the inserter 504 from the free state to the actuated state; moving the staple 502 from the free state to the elastically deformed state; moving the legs 512, 514 to be parallel; inserting the left leg 512 into the first hole; inserting the right leg 514 into the second hole; seating the bridge 506 against the first and/or second body part; moving the inserter 504 from the actuated state toward the free state; moving the staple 502 from the elastically deformed state toward the free state; moving the legs 512, 514 toward a converging state; rupturing the connections 538, 540; and removing the inserter 504 from the implantation site, leaving the staple 502 in place.

Making the first and/or second holes may involve drilling the first and/or second holes, optionally using a drill guide. The first hole may be substantially parallel to the second hole. The first and/or second body parts may be bone. Moving the inserter 504 from the free state to the actuated state may occur substantially in advance of a surgical procedure in which the delivery device 500 is used or immediately before inserting the legs 512, 514 into the first and second holes, respectively. Moving the inserter 504 from the free state to the actuated state may cause the staple 502 to move from the free state to the elastically deformed state. When the staple 502 is in the elastically deformed state, the left leg 512 may be substantially parallel to the right leg 514. Inserting the legs 512, 514 into the first and second holes, respectively, may involve partially or completely provisionally inserting the legs 512, 514 into the first and second holes and partially or completely withdrawing the legs 512, 514 from the first and second holes, while the inserter 504 is in the actuated state, and/or while the staple 502 is in the elastically deformed state, and/or before rupturing the connections 538, 540. Seating the bridge 506 against the first and/or second body part may involve direct contact between a bone facing side of the bridge 506 (a surface of the bridge 506 that faces towards the distal ends 518, 522) and a surface of the first and/or second body part. Moving the inserter 504 from the actuated state toward the free state may involve releasing pressure on the proximal portions 532, 536 acting to urge the proximal portions 532, 536 towards each other and towards the plane 525. Moving the inserter 504 from the actuated state toward the free state may cause the staple 502 to move from the elastically deformed state toward the free state. The inserter 504 and the staple 502 may relax toward their respective free states, but under certain circumstances they may not completely achieve their free states. Rupturing the connections may occur while the inserter 504 is in the actuated state and/or while the staple 502 is in the elastically deformed state. Rupturing the connections may cause the staple 502 to move from the elastically deformed state toward the free state.

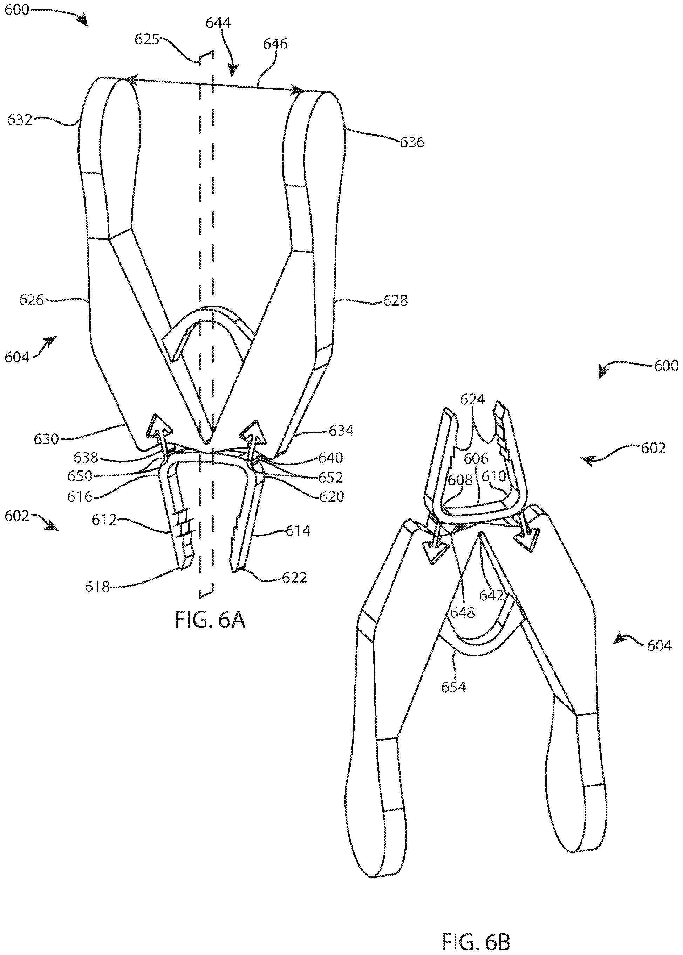

Referring to FIGS. 6A-6D, yet another delivery device 600 includes a staple 602 and an inserter 604. The delivery device 600 may be similar to the delivery device 500. However, in this example, the staple 602 and inserter 604 are formed separately and coupled together. The staple 602 may be made of a metal, such as a nickel titanium alloy, or a polymer such as PEEK or a polymeric composition based upon PEEK. The inserter 604 may be made of a metal or a polymer.

The staple 602 includes a bridge 606 which extends longitudinally between a left end 608 and a right end 610. The staple 602 includes a left leg 612 and a right leg 614. A proximal end 616 of the left leg 612 is attached to the left end 608 of the bridge 606. The left leg 612 terminates in a distal end 618, which is a free end opposite the bridge 606. A proximal end 620 of the right leg 614 is attached to the right end 610 of the bridge 606. The right leg 614 terminates in a distal end 622, which is a free end opposite the bridge 606. The facing surfaces of the legs 612, 614 include teeth 624. When the staple 602 is implanted, the teeth 624 help to retain the staple 602 in the desired position. The teeth 624 may be on any or all sides of the legs 612, 614.

The staple 602 includes left and right securing members 656, 658 which function as left and right connections 638, 640 very similar to the connections 538, 540. The left securing member 656 extends from the left end 608 of the bridge 606 opposite the left leg 612, and terminates in a left locking member 660. The right securing member 658 extends from the right end 610 of the bridge 606 opposite the right leg 614, and terminates in a right locking member 662. Each locking member 660, 662 is bilaterally enlarged in the left-right direction relative to its respective securing member 656, 658, so that bilateral undercuts are formed at the transition between the locking member and the securing member. The locking members 660, 662 are shaped like arrowheads in this example. There are notches 650 on both sides of the left securing member 656 at the connection with the left end 608 of the bridge 606. There are notches 652 on both sides of the right securing member 658 at the connection with the right end 610 of the bridge 606.

The staple 602 is shown in its free state, in which the staple 602 is not elastically or plastically deflected or deformed. The free state may be referred to as the closed state. In one example of the free state, the staple 602 experiences no external loads, other than gravity perhaps. However, in the free state, the staple 602 may experience loads that are below a threshold for elastic or plastic deflection or deformation. In the free state, the distal ends 618, 622 of the legs 612, 614 are closer together than the proximal ends 616, 620 so that the legs 612, 614 converge as they extend away from the bridge 606.

The staple 602 may be moved between the free state and an elastically deformed state by elastically deforming some or all of the staple 602. For example, elastically deflecting or pressing on the bridge 606 so that it is straight or curves down between the legs 612, 614 causes the distal ends 618, 622 to spread apart. The legs 612, 614 may spread apart in the elastically deformed state so as to become parallel. The elastically deformed state may be referred to as the open state. The elastically deformed state may be suitable for implantation of the staple 602 and may thus be referred to as an insertion state or an implantation state.

The staple 602 may be modified to include a reinforcing member as described herein. In this situation, the staple 602 may be referred to as a hybrid staple 602 because it includes a polymer base member and a metal reinforcing member. The staple 602 may be replaced by staple 802, described below.

The inserter 604 may be referred to as a delivery member. The inserter 604 includes a left body 626 and a right body 628 which in this example is a mirror image of the left body 626 across a first plane of symmetry 625 (FIG. 6A). The left body 626 includes a distal portion 630 and a proximal portion 632. The right body 628 includes a distal portion 634 and a proximal portion 636.

The left distal portion 630 includes a left recess 664 formed in a distal side of the body 626. The left recess 664 includes a wide portion 668 that is shaped to receive the left locking member 660 and a narrow portion 670 that is shaped to receive at least a portion of the left securing member 656. The right distal portion 634 includes a right recess 672 formed in a distal side of the body 628. The right recess 672 includes a wide portion 674 that is shaped to receive the right locking member 662 and a narrow portion 676 that is shaped to receive at least a portion of the right securing member 658. The recesses 664, 672 may be referred to as formations or pockets. The recesses 664, 672 engage the bilateral undercuts so that tension may be applied to the securing members 656,658 by the inserter 604. The recesses 664, 672 may receive the securing members 656, 658 and locking members 660, 662 with a close fit or an interference fit, and may even be formed around the securing members 656, 658 and locking members 660, 662 (or vice versa) in a molding operation.

The left distal portion 630 is connected to the left end 608 of the bridge 606 by the left connection 638 (securing member 656 and locking member 660) received in the recess 664. The right distal portion 634 is connected to the right end 610 of the bridge 606 by the right connection 640 received in the recess 672. The connections 638, 640 may slide into engagement with the recesses 664, 672 from the front or back of the inserter 604. An adhesive may be used. After insertion, the locking members 660, 662 cannot be easily removed from the inserter 604 until the surgeon decides that the staple 602 should be implanted. The distal portions 630, 634 are connected to the bridge 606 solely by the connections 638, 640, which are at or close to the ends 608, 610. The connections 638, 640 may be ruptured after the staple 602 has been implanted, to disconnect the inserter 604 from the staple 602. For example, the connections 638, 640 may be ruptured at the notches 650, 652 by use of a surgical wire cutter, scissors, a scalpel, or a rupturing instrument specifically designed to correspond to the connections 638, 640, or by being manually broken. After rupture, the bridge 606 may include residue from the ruptured connections 638, 640, such as residue from the ruptured securing members 656, 658. Preferably, the notches 650, 652 are positioned so that little or no residue protrudes from the bridge. The distal portions 630, 634 extend along and above the bridge 606 and are joined together at a central junction 642 which is shown in the vicinity of the plane 625. The central junction 642 is not secured to the bridge 606, but can be brought into contact with the bridge 606 as discussed below.

The inserter 604 includes a flex bridge 654 which extends between the bodies 626, 628. The flex bridge 654 is curved or bent so that the flex bridge 654 is deformable, preferably elastically deformable. The flex bridge 654 maintains the bodies 626, 628 in a desired spatial configuration. The flex bridge 654 may be integrally formed with the inserter 604, or it may be a separate part that is attached between the bodies 626, 628.

The inserter 604 is shown in its free state, in which the inserter 604 is not elastically or plastically deflected or deformed. In one example of the free state, the inserter 604 experiences no external loads, other than gravity perhaps. However, in the free state, the inserter 604 may experience loads that are below a threshold for elastic or plastic deflection or deformation. In the free state, the proximal portions 632, 636 are separated by a gap 644 having a free state dimension 646. In the free state, the central junction 642 may contact the bridge 606, or the central junction 642 may optionally be separated from the bridge by a gap 648.

The inserter 604 may be moved between the free state and an actuated state by moving or pressing the proximal portions 632, 636 towards each other and towards the plane 625, thereby changing the shape of the inserter 604. This can be done manually, for example with an index finger on the left proximal portion 632 and a thumb on the right proximal portion 636. In other examples, the proximal portions 632, 636 may be moved away from each other or otherwise moved relative to each other, to move the inserter 604 between the free state and the actuated state. The actuated state may be referred to as a compressed state, an insertion state, or an implantation state. When the inserter 604 is moved from the free state to the actuated state, the gap 644 becomes smaller, the bodies 626, 628 pivot about the connections 638, 640 respectively, the flex bridge 654 deforms, and the central junction 642 presses against the bridge 606, thus causing the staple 602 to move from the free state to the elastically deformed state. Conversely, as the inserter 604 is moved from the actuated state to the free state, the staple 602 moves from the elastically deformed state to the free state.

When the inserter 604 is in the actuated state and the staple 602 is in the elastically deformed state, the delivery device 600 may be used to implant the staple 602. After the staple 602 has been implanted, the connections 638, 640 may be ruptured. The inserter 604 may then be removed from the implantation site, leaving the staple 602 in place.

Methods of manufacturing the delivery device 600 may include: (1) forming the staple 602 from a first material; forming the inserter 604 from a second material that may be the same as or different from the first material; and securing the staple 602 and the inserter 604 together; (3) forming the staple 602 from the first material; and forming the inserter 604 from the second material in contact with the previously formed staple 602; or (4) forming the inserter 604 from the second material; and forming the staple 602 from the first material in contact with the previously formed inserter 604. Shaping may involve molding, cutting, additive manufacturing processes, or the like. Molding may involve injection molding, insert molding, over molding, or the like. Cutting may involve laser cutting. Additive manufacturing may involve three-dimensional printing.

A method of using the delivery device 600, not forming part of the claimed invention, may include any or all of the following steps in any order: connecting the staple 602 to the inserter 604; making a first hole in a first body part; making a second hole in a second body part; moving the inserter 604 from the free state to the actuated state; moving the staple 602 from the free state to the elastically deformed state; moving the legs 612, 614 to be parallel; inserting the left leg 612 into the first hole; inserting the right leg 614 into the second hole; seating the bridge 606 against the first and/or second body part; moving the inserter 604 from the actuated state toward the free state; moving the staple 602 from the elastically deformed state toward the free state; moving the legs 612, 614 toward a converging state; rupturing the connections 638, 640; and removing the inserter 604 from the implantation site, leaving the staple 602 in place.

Making the first and/or second holes may involve drilling the first and/or second holes, optionally using a drill guide. The first hole may be substantially parallel to the second hole. The first and/or second body parts may be bone. Moving the inserter 604 from the free state to the actuated state may occur substantially in advance of a surgical procedure in which the delivery device 600 is used or immediately before inserting the legs 612, 614 into the first and second holes, respectively. Moving the inserter 604 from the free state to the actuated state may cause the staple 602 to move from the free state to the elastically deformed state. When the staple 602 is in the elastically deformed state, the left leg 612 may be substantially parallel to the right leg 614. Inserting the legs 612, 614 into the first and second holes, respectively, may involve partially or completely provisionally inserting the legs 612, 614 into the first and second holes and partially or completely withdrawing the legs 612, 614 from the first and second holes, while the inserter 604 is in the actuated state, and/or while the staple 602 is in the elastically deformed state, and/or before rupturing the connections 638, 640. Seating the bridge 606 against the first and/or second body part may involve direct contact between a bone facing side of the bridge 606 (a surface of the bridge 606 that faces towards the distal ends 618, 622) and a surface of the first and/or second body part. Moving the inserter 604 from the actuated state toward the free state may involve releasing pressure on the proximal portions 632, 636 acting to urge the proximal portions 632, 636 towards each other and towards the plane 625. Moving the inserter 604 from the actuated state toward the free state may cause the staple 602 to move from the elastically deformed state toward the free state. The inserter 604 and the staple 602 may relax toward their respective free states, but under certain circumstances they may not completely achieve their free states. Rupturing the connections may occur while the inserter 604 is in the actuated state and/or while the staple 602 is in the elastically deformed state. Rupturing the connections may cause the staple 602 to move from the elastically deformed state toward the free state.

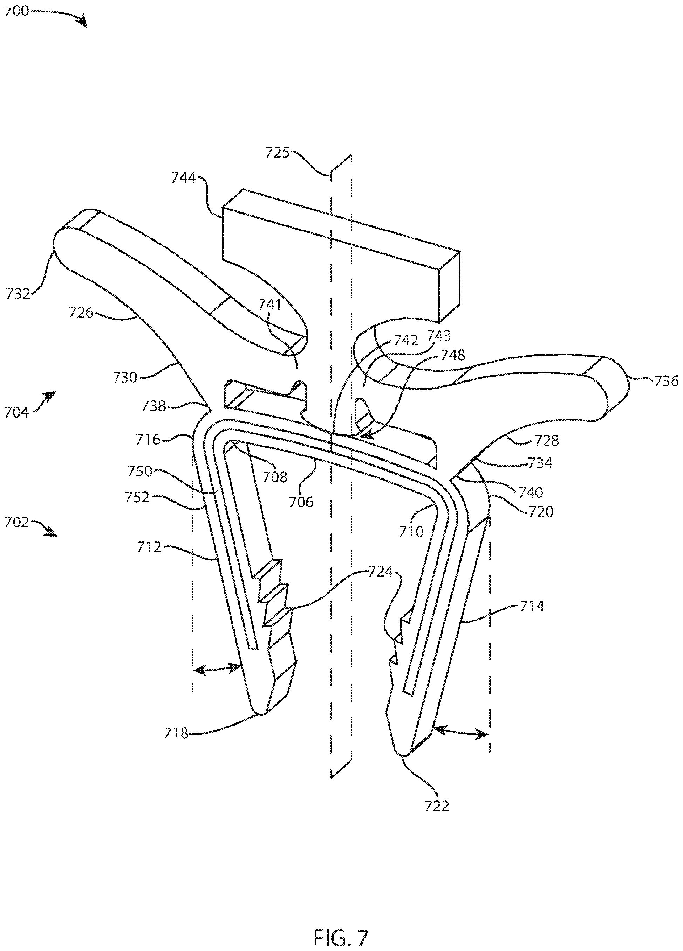

Referring to FIG. 7, yet another delivery device 700 includes a staple 702 and an inserter 704. In this example, the staple 702 and inserter 704 are integrally formed as a monolithic delivery device 700. The staple 702 and inserter 704 may be made of a polymer such as PEEK or a polymeric composition based upon PEEK. Alternatively, the staple 702 may be made of a first polymer and the inserter 704 may be made of a second polymer which is different from the first polymer. In this arrangement, the staple 702 and the inserter 704 may be coupled together to form the delivery device 700, for example by bonding, for example by melt bonding. The staple 702 and inserter 704 may also be made of metal, such as a nickel titanium alloy.

The staple 702 may be similar to the staple 302. The staple 702 includes a bridge 706 which extends longitudinally between a left end 708 and a right end 710. The staple 702 includes a left leg 712 and a right leg 714. A proximal end 716 of the left leg 712 is attached to the left end 708 of the bridge 706. The left leg 712 terminates in a distal end 718, which is a free end opposite the bridge 706. A proximal end 720 of the right leg 714 is attached to the right end 710 of the bridge 706. The right leg 714 terminates in a distal end 722, which is a free end opposite the bridge 706. The facing surfaces of the legs 712, 714 include teeth 724. When the staple 702 is implanted, the teeth 724 help to retain the staple 702 in the desired position. The teeth 724 may be on any or all sides of the legs 712, 714.

The staple 702 includes a reinforcing member 750 which may be metal, for example a nickel titanium alloy. The reinforcing member 750 may have a rectangular, square, or round cross section. The reinforcing member 750 may be easily fabricated from ribbon stock, square wire stock, or round wire stock. The reinforcing member 750 extends along the bridge 706 and optionally part way along the legs 712, 714. The reinforcing member 750 may extend along 50-90% of the length of each leg 712, 714. The reinforcing member 750 may be partially or wholly embedded in the bridge 706 and the legs 712, 714 of the staple 702, or it may be attached to the surface of the bridge 706 and the legs 712, 714. The staple 702 may include a base member 752, which in this example is the polymer portion of the staple 702 other than the reinforcing member 750. The base member 752 may be easily molded with the desired three-dimensional characteristics for the staple 702. The base member 752 may include a channel into which the reinforcing member 750 is fitted. The staple 702 may be referred to as a hybrid staple 702 because it includes a polymer base member 752 and a metal reinforcing member 750.

The staple 702 is shown in its free state, in which the staple 702 is not elastically or plastically deflected or deformed. The free state may be referred to as the closed state. In one example of the free state, the staple 702 experiences no external loads, other than gravity perhaps. However, in the free state, the staple 702 may experience loads that are below a threshold for elastic or plastic deflection or deformation. In the free state, the distal ends 718, 722 of the legs 712, 714 are closer together than the proximal ends 716, 720 so that the legs 712, 714 converge as they extend away from the bridge 706. The reinforcing member 750 is preferably in the form of a shallow upwards curve, convex on the proximal side and concave on the distal side facing the distal ends 718, 722. The reinforcing member 750 may maintain the legs 712, 714 in the free state.

The staple 702 may be moved between the free state and an elastically deformed state by elastically deforming some or all of the staple 702, including corresponding portions of the reinforcing member 750. For example, elastically deflecting or pressing on the bridge 706 so that it is straight or curves down between the legs 712, 714 causes the distal ends 718, 722 to spread apart. The legs 712, 714 may spread apart in the elastically deformed state so as to become parallel, as indicated by the dashed lines and movement arrows next to the legs 712, 714 in FIG. 7. The elastically deformed state may be referred to as the open state. The elastically deformed state may be suitable for implantation of the staple 702 and may thus be referred to as an insertion state or an implantation state.

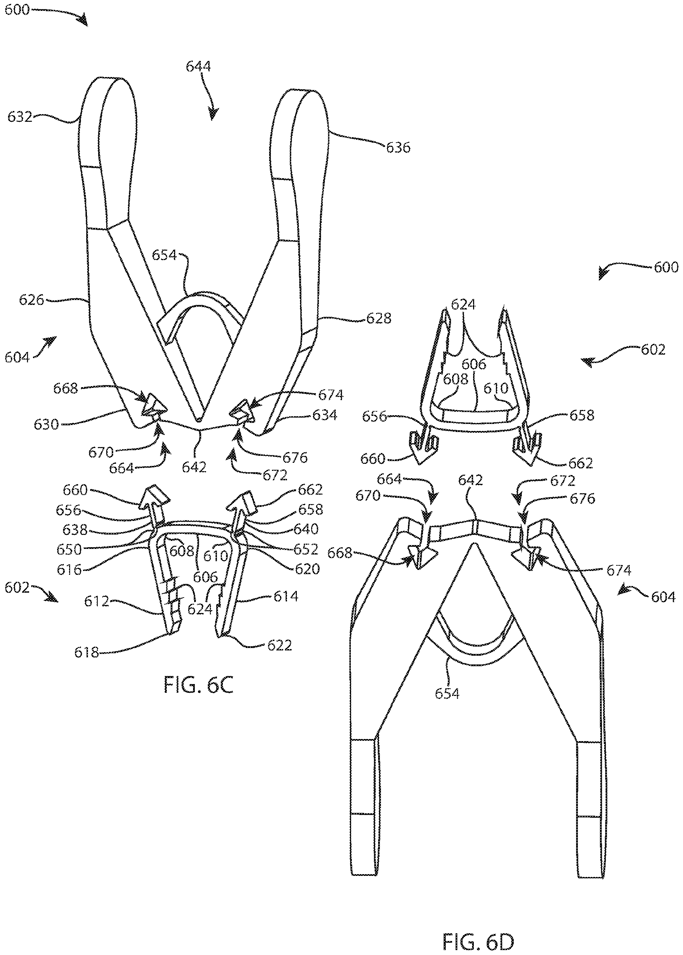

The inserter 704 may be referred to as a delivery member. The inserter 704 includes a left body 726 and a right body 728 which in this example is a mirror image of the left body 726 across a first plane of symmetry 725. The left body 726 includes a distal portion 730 and a proximal portion 732. The right body 728 includes a distal portion 734 and a proximal portion 736. The left distal portion 730 is connected to the left end 708 of the bridge 706 by a left connection 738. The right distal portion 734 is connected to the right end 710 of the bridge 706 by a right connection 740. The distal portions 730, 734 are connected to the bridge 706 solely by the connections 738, 740, which are at or close to the ends 708, 710. The connections 738, 740 may be ruptured after the staple 702 has been implanted, to disconnect the inserter 704 from the staple 702. For example, the connections 738, 740 may be ruptured by use of a surgical wire cutter, scissors, a scalpel, or a rupturing instrument specifically designed to correspond to the connections 738, 740, or by being manually broken. After rupture, the bridge 706 may include residue from the ruptured connections 738, 740. The integral connections 738, 740 shown may be replaced by the connections 538, 540 or the securing members 656, 658, locking members 660, 662, and recesses 664, 672 shown in FIG. 6 for delivery device 600. The distal portions 730, 734 extend along and above the bridge 706 and are joined together at a central junction 742 which is shown in the vicinity of the plane 725. The distal portion 730 is joined to the central junction 742 by a left flexible joint 741 and the distal portion 734 is joined to the central junction 742 by a right flexible joint 743. The central junction 742 is not secured to the bridge 706, but can be brought into contact with the bridge 706 as discussed below. A central component 744 extends proximally from the central junction 742 and becomes bilaterally enlarged in a left to right direction as it extends proximally. The proximal side of the central component 744 is flat so that pressure or force may be applied to it manually or with an instrument.

The inserter 704 is shown in its free state, in which the inserter 704 is not elastically or plastically deflected or deformed. In one example of the free state, the inserter 704 experiences no external loads, other than gravity perhaps. However, in the free state, the inserter 704 may experience loads that are below a threshold for elastic or plastic deflection or deformation. In the free state, the central junction 742 may contact the bridge 706, or the central junction 742 may optionally be separated from the bridge by a gap 748.

The inserter 704 may be moved between the free state and an actuated state by moving or pressing the proximal portions 732, 736 towards each other and towards the plane 725 and by moving or pressing the central component 744 distally, thereby changing the shape of the inserter 704. This can be done manually, for example with an index finger on the distal aspect of the left proximal portion 732, a middle finger on the distal aspect of the right proximal portion 736, and a thumb on the proximal aspect of the central component 744. In other examples, the proximal portions 732, 736 may be moved away from each other or otherwise moved relative to each other, to move the inserter 704 between the free state and the actuated state. The actuated state may be referred to as a compressed state, an insertion state, or an implantation state. When the inserter 704 is moved from the free state to the actuated state, the bodies 726, 728 pivot towards each other and towards the first plane of symmetry about the connections 738, 740 respectively, and the central junction 742 presses against the bridge 706, thus causing the staple 702 to move from the free state to the elastically deformed state. Conversely, as the inserter 704 is moved from the actuated state to the free state, the staple 702 moves from the elastically deformed state to the free state. The reinforcing member 750 may urge the base member 752 toward the free state and may increase the post-implantation compressive force of the staple 702 compared to unreinforced polymer staples.

When the inserter 704 is in the actuated state and the staple 702 is in the elastically deformed state, the delivery device 700 may be used to implant the staple 702. After the staple 702 has been implanted, the connections 738, 740 may be ruptured. The inserter 704 may then be removed from the implantation site, leaving the staple 702 in place.

Methods of manufacturing the delivery device 700 may include: (1) forming the base member 752 and the inserter 704 as a monolithic article by shaping a single material in a single step; forming the reinforcing member 750 from metal, preferably a nickel titanium alloy; and connecting the reinforcing member 750 to the base member 752; (2) forming the base member 752 from a first material; forming the inserter 704 from a second material that may be the same as or different from the first material; forming the reinforcing member 750 from a third material that is metal, preferably a nickel titanium alloy; and securing the base member 752, the inserter 704, and the reinforcing member 750 together; (3) forming the reinforcing member 750 from the third material; forming the base member 752 from the first material in contact with the previously formed reinforcing member 750 to form the staple 702; and forming the inserter 704 from the second material in contact with the previously formed staple 702; or (4) forming the inserter 704 from the second material; forming the reinforcing member 750 from the third material; and forming the base member 752 from the first material in contact with the previously formed inserter 704 and reinforcing member 750. The steps of forming the base member 352 and the inserter 704 as a monolithic article and connecting the reinforcing member 750 may occur simultaneously, for example as an overmolding or insert molding operation. Shaping may involve molding, cutting, additive manufacturing processes, or the like. Molding may involve injection molding, insert molding, over molding, or the like. Cutting may involve laser cutting. Additive manufacturing may involve three-dimensional printing.

A method of using the delivery device 700, not forming part of the claimed invention, may include any or all of the following steps in any order: making a first hole in a first body part; making a second hole in a second body part; moving the inserter 704 from the free state to the actuated state; moving the staple 702 from the free state to the elastically deformed state; moving the legs 712, 714 to be parallel; inserting the left leg 712 into the first hole; inserting the right leg 714 into the second hole; seating the bridge 706 against the first and/or second body part; moving the inserter 704 from the actuated state toward the free state; moving the staple 702 from the elastically deformed state toward the free state; moving the legs 712, 714 toward a converging state; rupturing the connections 738, 740; and removing the inserter 704 from the implantation site, leaving the staple 702 in place.

Making the first and/or second holes may involve drilling the first and/or second holes, optionally using a drill guide. The first hole may be substantially parallel to the second hole. The first and/or second body parts may be bone. Moving the inserter 704 from the free state to the actuated state may occur substantially in advance of a surgical procedure in which the delivery device 700 is used or immediately before inserting the legs 712, 714 into the first and second holes, respectively. Moving the inserter 704 from the free state to the actuated state may cause the staple 702 to move from the free state to the elastically deformed state. When the staple 702 is in the elastically deformed state, the left leg 712 may be substantially parallel to the right leg 714. Inserting the legs 712, 714 into the first and second holes, respectively, may involve partially or completely provisionally inserting the legs 712, 714 into the first and second holes and partially or completely withdrawing the legs 712, 714 from the first and second holes, while the inserter 704 is in the actuated state, and/or while the staple 702 is in the elastically deformed state, and/or before rupturing the connections 738, 740. Seating the bridge 706 against the first and/or second body part may involve direct contact between a bone facing side of the bridge 706 (a surface of the bridge 706 that faces towards the distal ends 718, 722) and a surface of the first and/or second body part. Moving the inserter 704 from the actuated state toward the free state may involve releasing pressure on the proximal portions 732, 736 acting to urge the proximal portions 732, 736 towards each other and towards the plane 725. Moving the inserter 704 from the actuated state toward the free state may cause the staple 702 to move from the elastically deformed state toward the free state. The inserter 704 and the staple 702 may relax toward their respective free states, but under certain circumstances they may not completely achieve their free states. Rupturing the connections may occur while the inserter 704 is in the actuated state and/or while the staple 702 is in the elastically deformed state. Rupturing the connections may cause the staple 702 to move from the elastically deformed state toward the free state.

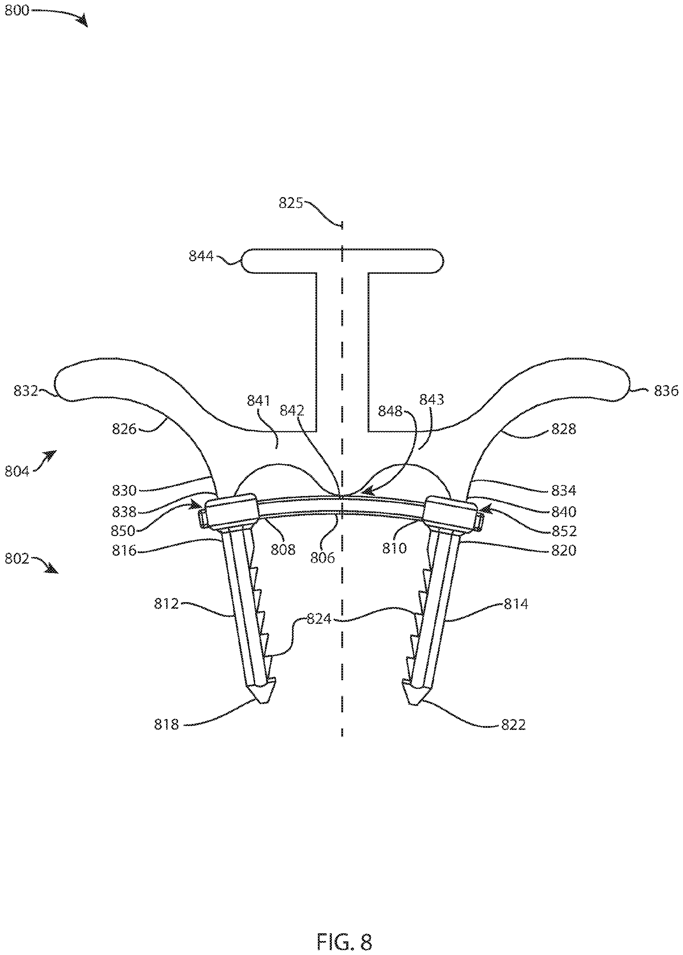

Referring to FIG. 8, yet another delivery device 800 includes a staple 802 and an inserter 804. In this example, the inserter 804 and at least part of the staple 802 are integrally formed. The staple 802 and inserter 804 may be made of a polymer such as PEEK or a polymeric composition based upon PEEK. Alternatively, the staple 802 may be made of a first polymer and the inserter 804 may be made of a second polymer which is different from the first polymer. In this arrangement, the staple 802 and the inserter 804 may be coupled together to form the delivery device 800, for example by bonding, for example by melt bonding. The staple 802 and inserter 804 may also be made of metal.

The staple 802 includes a bridge 806 which extends longitudinally between a left end 808 and a right end 810. The staple 802 includes a left leg 812 and a right leg 814. A proximal end 816 of the left leg 812 is attached to the left end 808 of the bridge 806. The left leg 812 terminates in a distal end 818, which is a free end opposite the bridge 806. A proximal end 820 of the right leg 814 is attached to the right end 810 of the bridge 806. The right leg 814 terminates in a distal end 822, which is a free end opposite the bridge 806. The facing surfaces of the legs 812, 814 include teeth 824. When the staple 802 is implanted, the teeth 824 help to retain the staple 802 in the desired position. The teeth 824 may be on any or all sides of the legs 812, 814.

The staple 802 in this example is a three piece assembly of a metal bridge 806 and polymer legs 812, 814. The bridge 806 may be a nickel titanium alloy and the legs 812, 814 may be PEEK or a polymer composition based on PEEK. In other examples, the bridge 806 may be a first material, metal or polymer, and the legs 812, 814 may be a second material, metal or polymer, which is the same as or different from the first material. The metal bridge 806 may function similar to the reinforcing members described herein. The proximal ends 816, 820 of the legs 812, 814 are bilaterally enlarged at least in a left to right direction, and optionally in a front to back direction, relative to their respective distal ends 818, 822. The proximal end 816 includes a socket 850 which receives the left end 808 of the bridge 806 and the proximal end 820 includes a socket 852 which receives the right end 810. The staple 802 may be referred to as a hybrid staple 802 because it includes polymer legs 812, 814 and a metal bridge 806. The three piece assembly of a metal bridge and polymer legs may be incorporated in any of the staples 102, 302, 502, 602, 702, 900, 1100, 1800, 1400, 1500, 1600 disclosed herein.

The staple 802 is shown in its free state, in which the staple 802 is not elastically or plastically deflected or deformed. The free state may be referred to as the closed state. In one example of the free state, the staple 802 experiences no external loads, other than gravity perhaps. However, in the free state, the staple 802 may experience loads that are below a threshold for elastic or plastic deflection or deformation. In the free state, the distal ends 818, 822 of the legs 812, 814 are closer together than the proximal ends 816, 820 so that the legs 812, 814 converge as they extend away from the bridge 806. The bridge 806 is preferably in the form of a very shallow upwards curve, convex on the proximal side and concave on the distal side facing the distal ends 818, 822. The bridge 806 may maintain the legs 812, 814 in the free state.

The staple 802 may be moved between the free state and an elastically deformed state by elastically deforming some or all of the staple 802, including portions of the bridge 806. For example, elastically deflecting or pressing on the bridge 806 so that it is straight or curves down between the legs 812, 814 causes the distal ends 818, 822 to spread apart. The legs 812, 814 may spread apart in the elastically deformed state so as to become parallel. The elastically deformed state may be referred to as the open state. The elastically deformed state may be suitable for implantation of the staple 802 and may thus be referred to as an insertion state or an implantation state.

The inserter 804 may be similar to the inserter 704. The inserter 804 may be referred to as a delivery member. The inserter 804 includes a left body 826 and a right body 828 which in this example is a mirror image of the left body 826 across a first plane of symmetry 825, which is shown edge on in FIG. 8 and is therefore represented by a line 825. The left body 826 includes a distal portion 830 and a proximal portion 832. The right body 828 includes a distal portion 834 and a proximal portion 836. The left distal portion 830 is connected to the proximal end 816 of the left leg 812 by a left connection 838. The right distal portion 834 is connected to the proximal end 820 of the right leg 814 by a right connection 840. The distal portions 830, 834 are connected to the legs 812, 814, and thus to the bridge 806, solely by the connections 838, 840, which are at or close to the ends 808, 810. The connections 838, 840 may be ruptured after the staple 802 has been implanted, to disconnect the inserter 804 from the staple 802. For example, the connections 838, 840 may be ruptured by use of a surgical wire cutter, scissors, a scalpel, or a rupturing instrument specifically designed to correspond to the connections 838, 840, or by being manually broken. After rupture, the proximal ends 816, 820 may include residue from the ruptured connections 838, 840. The connections 838, 840 shown may be replaced by the connections 538, 540 or the securing members 656, 658, locking members 660, 662, and recesses 664, 672 shown in FIG. 6 for delivery device 600. The distal portions 830, 834 extend along and above the bridge 806 and are joined together at a central junction 842 which is shown in the vicinity of the plane 825. The distal portion 830 is joined to the central junction 842 by a left flexible joint 841 and the distal portion 834 is joined to the central junction 842 by a right flexible joint 843. The central junction 842 is not secured to the bridge 806, but can be brought into contact with the bridge 806 as discussed below. A central component 844 extends proximally from the central junction 842 and becomes bilaterally enlarged in a left to right direction as it extends proximally. The proximal side of the central component 844 is flat so that pressure or force may be applied to it manually or with an instrument.

The inserter 804 is shown in its free state, in which the inserter 804 is not elastically or plastically deflected or deformed. In one example of the free state, the inserter 804 experiences no external loads, other than gravity perhaps. However, in the free state, the inserter 804 may experience loads that are below a threshold for elastic or plastic deflection or deformation. In the free state, the central junction 842 may contact the bridge 806, or the central junction 842 may optionally be separated from the bridge by a gap 848.

The inserter 804 may be moved between the free state and an actuated state by moving or pressing the proximal portions 832, 836 towards each other and towards the plane 825 and by moving or pressing the central component 844 distally, thereby changing the shape of the inserter 804. This can be done manually, for example with an index finger on the distal aspect of the left proximal portion 832, a middle finger on the distal aspect of the right proximal portion 836, and a thumb on the proximal aspect of the central component 844. In other examples, the proximal portions 832, 836 may be moved away from each other or otherwise moved relative to each other, to move the inserter 804 between the free state and the actuated state. The actuated state may be referred to as a compressed state, an insertion state, or an implantation state. When the inserter 804 is moved from the free state to the actuated state, the bodies 826, 828 pivot towards each other and towards the first plane of symmetry about the connections 838, 840 respectively, and the central junction 842 presses against the bridge 806, thus causing the staple 802 to move from the free state to the elastically deformed state. Conversely, as the inserter 804 is moved from the actuated state to the free state, the staple 802 moves from the elastically deformed state to the free state. The metal bridge 806 may urge the staple 802 toward the free state and may increase the post-implantation compressive force of the staple 802 compared to an all-polymer staple.

When the inserter 804 is in the actuated state and the staple 802 is in the elastically deformed state, the delivery device 800 may be used to implant the staple 802. After the staple 802 has been implanted, the connections 838, 840 may be ruptured. The inserter 804 may then be removed from the implantation site, leaving the staple 802 in place.

The inserter 804 may be replaced by one of the inserters 104, 504, or 604.

Methods of manufacturing the delivery device 800 may include: (1) forming the legs 812, 814 and the inserter 804 as a monolithic article by shaping a single material in a single step; forming the bridge 806 of metal, preferably a nickel titanium alloy; and connecting the bridge 806 to the legs 812, 814; (2) forming the legs 812, 814 from a first material; forming the inserter 804 from a second material that may be the same as or different from the first material; forming the legs 812, 814 from a third material that is metal, preferably a nickel titanium alloy; and securing the legs 812, 814, the inserter 804, and the bridge 806 together; (3) forming the bridge 806 from the third material; forming the legs 812, 814 from the first material in contact with the previously formed bridge 806 to form the staple 802; and forming the inserter 804 from the second material in contact with the previously formed staple 802; or (4) forming the inserter 804 from the second material; forming the bridge 806 from the third material; and forming the legs 812, 814 from the first material in contact with the previously formed inserter 804 and bridge 806. The steps of forming the legs 812, 814 and the inserter 804 as a monolithic article and connecting the bridge 806 may occur simultaneously, for example as an overmolding or insert molding operation. Shaping may involve molding, cutting, additive manufacturing processes, or the like. Molding may involve injection molding, insert molding, over molding, or the like. Cutting may involve laser cutting. Additive manufacturing may involve three-dimensional printing.

A method of using the delivery device 800, not forming part of the claimed invention, may include any or all of the following steps in any order: making a first hole in a first body part; making a second hole in a second body part; moving the inserter 804 from the free state to the actuated state; moving the staple 802 from the free state to the elastically deformed state; moving the legs 812, 814 to be parallel; inserting the left leg 812 into the first hole; inserting the right leg 814 into the second hole; seating the bridge 806 against the first and/or second body part; moving the inserter 804 from the actuated state toward the free state; moving the staple 802 from the elastically deformed state toward the free state; moving the legs 812, 814 toward a converging state; rupturing the connections 838, 840; and removing the inserter 804 from the implantation site, leaving the staple 802 in place.

Making the first and/or second holes may involve drilling the first and/or second holes, optionally using a drill guide. The first hole may be substantially parallel to the second hole. The first and/or second body parts may be bone. Moving the inserter 804 from the free state to the actuated state may occur substantially in advance of a surgical procedure in which the delivery device 800 is used or immediately before inserting the legs 812, 814 into the first and second holes, respectively. Moving the inserter 804 from the free state to the actuated state may cause the staple 802 to move from the free state to the elastically deformed state. When the staple 802 is in the elastically deformed state, the left leg 812 may be substantially parallel to the right leg 814. Inserting the legs 812, 814 into the first and second holes, respectively, may involve partially or completely provisionally inserting the legs 812, 814 into the first and second holes and partially or completely withdrawing the legs 812, 814 from the first and second holes, while the inserter 804 is in the actuated state, and/or while the staple 802 is in the elastically deformed state, and/or before rupturing the connections 838, 840. Seating the bridge 806 against the first and/or second body part may involve direct contact between a bone facing side of the bridge 806 (a surface of the bridge 806 that faces towards the distal ends 818, 822) and a surface of the first and/or second body part. Moving the inserter 804 from the actuated state toward the free state may involve releasing pressure on the proximal portions 832, 836 acting to urge the proximal portions 832, 836 towards each other and towards the plane 825. Moving the inserter 804 from the actuated state toward the free state may cause the staple 802 to move from the elastically deformed state toward the free state. The inserter 804 and the staple 802 may relax toward their respective free states, but under certain circumstances they may not completely achieve their free states. Rupturing the connections may occur while the inserter 804 is in the actuated state and/or while the staple 802 is in the elastically deformed state. Rupturing the connections may cause the staple 802 to move from the elastically deformed state toward the free state.

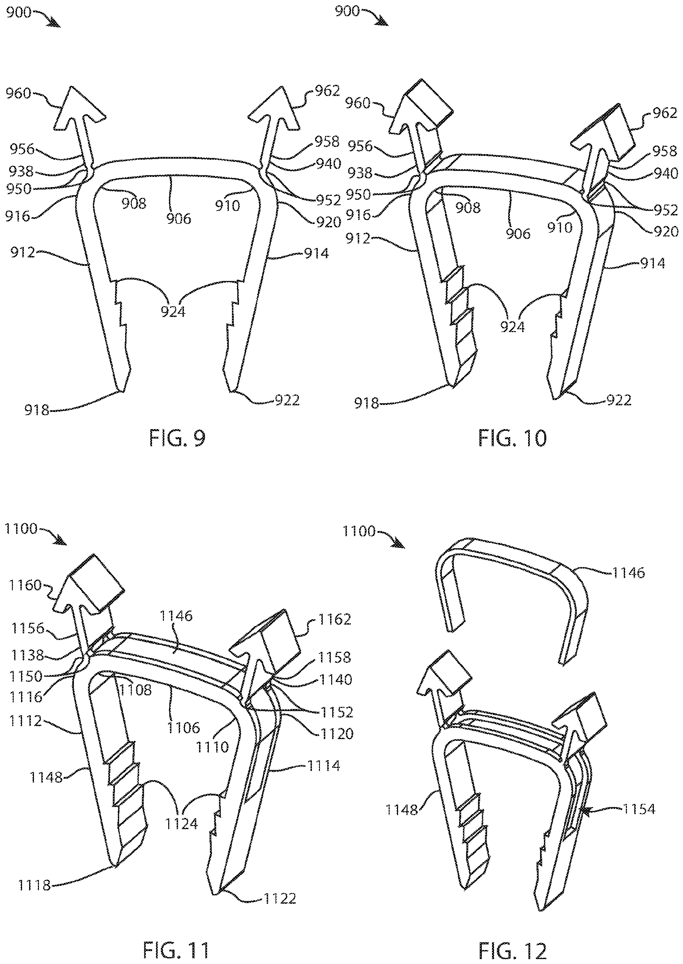

Referring to FIGS. 9 and 10, a staple 900 may be similar to, or identical to, staple 602 of delivery device 600. The staple 900 may be made of a metal, such as a nickel-titanium alloy, or a polymer such as PEEK or a polymeric composition based on PEEK. The staple 900, whether metal or polymer, may connect to inserter 604 in the same way as staple 602.

The staple 900 includes a bridge 906 which extends longitudinally between a left end 908 and a right end 910. The staple 900 includes a left leg 912 and a right leg 914. A proximal end 916 of the left leg 912 is attached to the left end 908 of the bridge 906. The left leg 912 terminates in a distal end 918, which is a free end opposite the bridge 906. A proximal end 920 of the right leg 914 is attached to the right end 910 of the bridge 906. The right leg 914 terminates in a distal end 922, which is a free end opposite the bridge 906. The facing surfaces of the legs 912, 914 include teeth 924. When the staple 900 is implanted, the teeth 924 help to retain the staple 900 in the desired position. The teeth 924 may be on any or all sides of the legs 912, 914.

The staple 900 includes left and right securing members 956, 958 which function as left and right connections 938, 940 very similar to the connections 538, 540. The left securing member 956 extends from the left end 908 of the bridge 906 opposite the left leg 912, and terminates in a left locking member 960. The right securing member 958 extends from the right end 910 of the bridge 906 opposite the right leg 914, and terminates in a right locking member 962. Each locking member 960, 962 is bilaterally enlarged in the left-right direction relative to its respective securing member 956, 958, so that bilateral undercuts are formed at the transition between the locking member and the securing member. The locking members 960, 962 are shaped like arrowheads in this example. There are notches 950 on both sides of the left securing member 956 at the connection with the left end 908 of the bridge 906. There are notches 952 on both sides of the right securing member 958 at the connection with the right end 910 of the bridge 906.

The staple 900 is shown in its free state, in which the staple 900 is not elastically or plastically deflected or deformed. The free state may be referred to as the closed state. In one example of the free state, the staple 900 experiences no external loads, other than gravity perhaps. However, in the free state, the staple 900 may experience loads that are below a threshold for elastic or plastic deflection or deformation. In the free state, the distal ends 918, 922 of the legs 912, 914 are closer together than the proximal ends 916, 920 so that the legs 912, 914 converge as they extend away from the bridge 906.

The staple 900 may be moved between the free state and an elastically deformed state by elastically deforming some or all of the staple 900. For example, elastically deflecting or pressing on the bridge 906 so that it is straight or curves down between the legs 912, 914 causes the distal ends 918, 922 to spread apart. The legs 912, 914 may spread apart in the elastically deformed state so as to become parallel. The elastically deformed state may be referred to as the open state. The elastically deformed state may be suitable for implantation of the staple 900 and may thus be referred to as an insertion state or an implantation state.

Referring to FIGS. 11 and 12, another staple 1100 may be similar to, or substantially identical to, staple 602 of delivery device 500. Staple 1100 may connect to inserter 604 in the same way as staple 602. Staple 1100 may be made of a polymer such as PEEK or a polymeric composition based on PEEK.

The staple 1100 includes a bridge 1106 which extends longitudinally between a left end 1108 and a right end 1110. The staple 1100 includes a left leg 1112 and a right leg 1114. A proximal end 1116 of the left leg 1112 is attached to the left end 1108 of the bridge 1106. The left leg 1112 terminates in a distal end 1118, which is a free end opposite the bridge 1106. A proximal end 1120 of the right leg 1114 is attached to the right end 1110 of the bridge 1106. The right leg 1114 terminates in a distal end 1122, which is a free end opposite the bridge 1106. The facing surfaces of the legs 1112, 1114 include teeth 1124. When the staple 1100 is implanted, the teeth 1124 help to retain the staple 1100 in the desired position. The teeth 1124 may be on any or all sides of the legs 1112, 1114.

The staple 1100 includes a reinforcing member 1146 which may be metal, for example a nickel titanium alloy. The reinforcing member 1146 may have a rectangular, square, or round cross section. The reinforcing member 1146 may be easily fabricated from ribbon stock, square wire stock, or round wire stock. The example shown has a rectangular cross section and is made from ribbon stock. The reinforcing member 1146 extends along the bridge 1106 and optionally part way along the legs 1112, 1114. The reinforcing member 1146 may extend along 50-90% of the length of each leg 1112, 1114. The reinforcing member 1146 may be partially or wholly embedded in the bridge 1106 and the legs 1112, 1114 of the staple 1100, or it may be attached to the surface of the bridge 1106 and the legs 1112, 1114. The staple 1100 may include a base member 1148, which in this example is the polymer portion of the staple 1100 other than the reinforcing member 1146. The base member 1148 may be easily molded with the desired three-dimensional characteristics for the staple 1100. The base member 1148 is shown with a channel 1154 that receives the reinforcing member 1146. The staple 1100 may be referred to as a hybrid staple 1100 because it includes a polymer base member 1148 and a metal reinforcing member 1146.

The staple 1100 includes left and right securing members 1156, 1158 which function as left and right connections 1138, 1140 very similar to the connections 538, 540. In this example, the securing members 1156, 1158 are integrally formed with the base member 1148, although they could be carried by the reinforcing member in other examples. The left securing member 1156 extends from the left end 1108 of the bridge 1106 opposite the left leg 1112, and terminates in a left locking member 1160. The right securing member 1158 extends from the right end 1110 of the bridge 1106 opposite the right leg 1114, and terminates in a right locking member 1162. Each locking member 1160, 1162 is bilaterally enlarged in the left-right direction relative to its respective securing member 1156, 1158, so that bilateral undercuts are formed at the transition between the locking member and the securing member. The locking members 1160, 1162 are shaped like arrowheads in this example. There are notches 1150 on both sides of the left securing member 1156 at the connection with the left end 1108 of the bridge 1106. There are notches 1152 on both sides of the right securing member 1158 at the connection with the right end 1110 of the bridge 1106.

The staple 1100 is shown in its free state, in which the staple 1100 is not elastically or plastically deflected or deformed. The free state may be referred to as the closed state. In one example of the free state, the staple 1100 experiences no external loads, other than gravity perhaps. However, in the free state, the staple 1100 may experience loads that are below a threshold for elastic or plastic deflection or deformation. In the free state, the distal ends 1118, 1122 of the legs 1112, 1114 are closer together than the proximal ends 1116, 1120 so that the legs 1112, 1114 converge as they extend away from the bridge 1106. The reinforcing member 1146 may maintain the legs 1112, 1114 in the free state.

The staple 1100 may be moved between the free state and an elastically deformed state by elastically deforming some or all of the staple 1100. For example, elastically deflecting or pressing on the bridge 1106 so that it is straight or curves down between the legs 1112, 1114 causes the distal ends 1118, 1122 to spread apart. The legs 1112, 1114 may spread apart in the elastically deformed state so as to become parallel. The elastically deformed state may be referred to as the open state. The elastically deformed state may be suitable for implantation of the staple 1100 and may thus be referred to as an insertion state or an implantation state. The reinforcing member 1146 may urge the base member 1148 from the elastically deformed state toward the free state and may increase the post-implantation compressive force of the staple 1100 compared to unreinforced polymer staples.

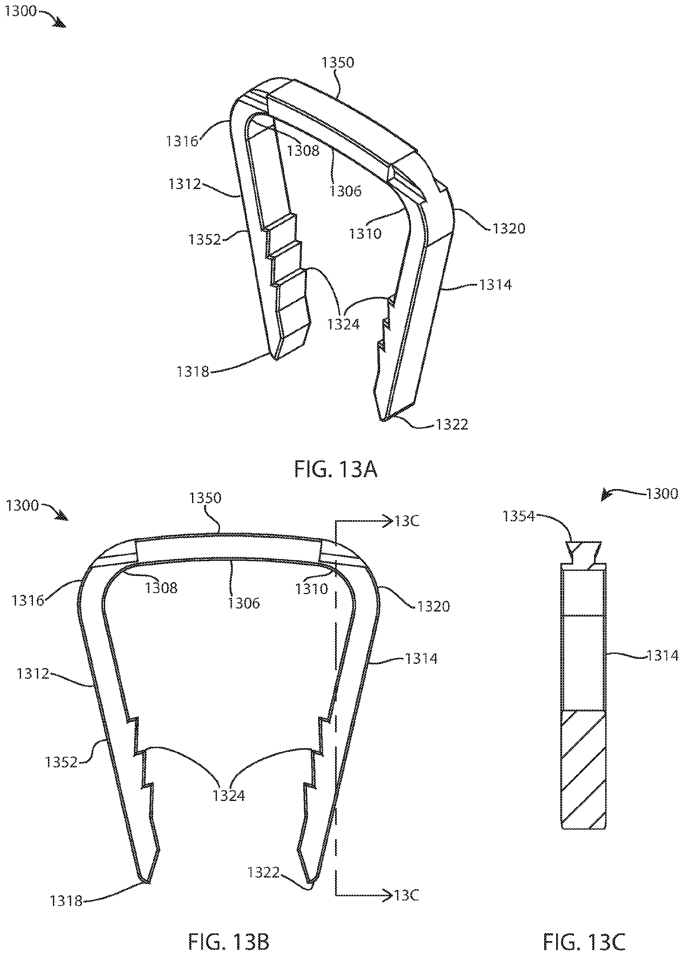

Referring to FIGS. 13A-13C, yet another staple 1300, not forming part of the claimed invention, includes a bridge 1306 which extends longitudinally between a left end 1308 and a right end 1310. The staple 1300 includes a left leg 1312 and a right leg 1314. A proximal end 1316 of the left leg 1312 is attached to the left end 1308 of the bridge 1306. The left leg 1312 terminates in a distal end 1318, which is a free end opposite the bridge 1306. A proximal end 1320 of the right leg 1314 is attached to the right end 1310 of the bridge 1306. The right leg 1314 terminates in a distal end 1322, which is a free end opposite the bridge 1306. The facing surfaces of the legs 1312, 1314 include teeth 1324. When the staple 1300 is implanted, the teeth 1324 help to retain the staple 1300 in the desired position. The teeth 1324 may be on any or all sides of the legs 1312, 1314.

The staple 1300 includes a reinforcing member 1350 which may be metal, for example a nickel titanium alloy. The reinforcing member 1350 in this example has a C-shaped cross section and thus forms a channel that is open on its distal side facing the distal ends 1318, 1322. The reinforcing member 1350 extends along the bridge 1306. The reinforcing member 1350 may be partially or wholly embedded in the bridge 1306 and the legs 1312, 1314 of the staple 1300, or it may be attached to the surface of the bridge 1306 and the legs 1312, 1314. In the example, the reinforcing member 1350 is attached to the front, proximal, and back surfaces of the bridge 1306 by a dovetail connection, although other undercut sliding connections are contemplated. The staple 1300 may include a base member 1352, which in this example is the polymer portion of the staple 1300 other than the reinforcing member 1350. The base member 1352 may be made of PEEK or a polymeric composition based on PEEK. The base member 1352 may be easily molded with the desired three-dimensional characteristics for the staple 1300. Referring to FIG. 13C, the base member 1352 includes a dovetail rail 1354 and the reinforcing member 1350 includes a complementary close-fitting dovetail channel. The staple 1300 may be referred to as a hybrid staple 1300 because it includes a polymer base member 1352 and a metal reinforcing member 1350.

The staple 1300 is shown in its free state, in which the staple 1300 is not elastically or plastically deflected or deformed. The free state may be referred to as the closed state. In one example of the free state, the staple 1300 experiences no external loads, other than gravity perhaps. However, in the free state, the staple 1300 may experience loads that are below a threshold for elastic or plastic deflection or deformation. In the free state, the distal ends 1318, 1322 of the legs 1312, 1314 are closer together than the proximal ends 1316, 1320 so that the legs 1312, 1314 converge as they extend away from the bridge 1306. The reinforcing member 1350 may maintain the legs 1312, 1314 in the free state.

The staple 1300 may be moved between the free state and an elastically deformed state by elastically deforming some or all of the staple 1300, including corresponding portions of the reinforcing member 1350. For example, elastically deflecting or pressing on the bridge 1306 so that it is straight or curves down between the legs 1312, 1314 causes the distal ends 1318, 1322 to spread apart. The legs 1312, 1314 may spread apart in the elastically deformed state so as to become parallel. The elastically deformed state may be referred to as the open state. The elastically deformed state may be suitable for implantation of the staple 1300 and may thus be referred to as an insertion state or an implantation state. The reinforcing member 1350 may urge the base member 1352 from the elastically deformed state toward the free state and may increase the post-implantation compressive force of the staple 1300 compared to unreinforced polymer staples.

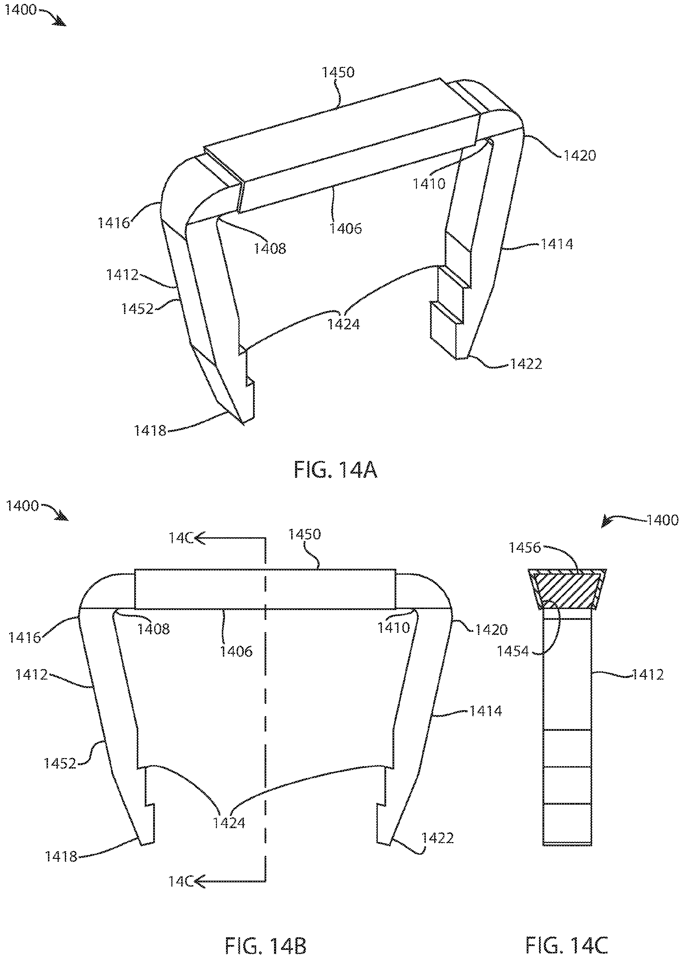

Referring to FIGS. 14A-14C, yet another staple 1400, not forming part of the claimed invention, may be similar to staple 1300.
Staple 1400 includes a bridge 1406 which extends longitudinally between a left end 1408 and a right end 1410. The staple 1400 includes a left leg 1412 and a right leg 1414. A proximal end 1416 of the left leg 1412 is attached to the left end 1408 of the bridge 1406. The left leg 1412 terminates in a distal end 1418, which is a free end opposite the bridge 1406. A proximal end 1420 of the right leg 1414 is attached to the right end 1410 of the bridge 1406. The right leg 1414 terminates in a distal end 1422, which is a free end opposite the bridge 1406. The facing surfaces of the legs 1412, 1414 include teeth 1424. When the staple 1400 is implanted, the teeth 1424 help to retain the staple 1400 in the desired position. The teeth 1424 may be on any or all sides of the legs 1412, 1414.

The staple 1400 includes a reinforcing member 1450 which may be metal, for example a nickel titanium alloy. The reinforcing member 1450 in this example has a C-shaped cross section and thus forms a channel that is open on its distal side facing the distal ends 1418, 1422. The reinforcing member 1450 extends along the bridge 1406. The reinforcing member 1450 may be partially or wholly embedded in the bridge 1406 and the legs 1412, 1414 of the staple 1400, or it may be attached to the surface of the bridge 1406 and the legs 1412, 1414. In the example, the reinforcing member 1450 is attached to the front, proximal, and back surfaces of the bridge 1406 by a dovetail connection, although other undercut sliding connections are contemplated. The staple 1400 may include a base member 1452, which in this example is the polymer portion of the staple 1400 other than the reinforcing member 1450. The base member 1452 may be made of PEEK or a polymeric composition based on PEEK. The base member 1452 may be easily molded with the desired three-dimensional characteristics for the staple 1400. Referring to FIG. 14C, the base member 1452 includes a dovetail rail 1454 and the reinforcing member 1450 includes a complementary close-fitting dovetail channel 1456. The staple 1400 may be referred to as a hybrid staple 1400 because it includes a polymer base member 1452 and a metal reinforcing member 1450.

The staple 1400 is shown in its free state, in which the staple 1400 is not elastically or plastically deflected or deformed. The free state may be referred to as the closed state. In one example of the free state, the staple 1400 experiences no external loads, other than gravity perhaps. However, in the free state, the staple 1400 may experience loads that are below a threshold for elastic or plastic deflection or deformation. In the free state, the distal ends 1418, 1422 of the legs 1412, 1414 are closer together than the proximal ends 1416, 1420 so that the legs 1412, 1414 converge as they extend away from the bridge 1406. The reinforcing member 1450 may maintain the legs 1412, 1414 in the free state.

The staple 1400 may be moved between the free state and an elastically deformed state by elastically deforming some or all of the staple 1400, including corresponding portions of the reinforcing member 1450. For example, elastically deflecting or pressing on the bridge 1406 so that it is straight or curves down between the legs 1412, 1414 causes the distal ends 1418, 1422 to spread apart. The legs 1412, 1414 may spread apart in the elastically deformed state so as to become parallel. The elastically deformed state may be referred to as the open state. The elastically deformed state may be suitable for implantation of the staple 1400 and may thus be referred to as an insertion state or an implantation state. The reinforcing member 1450 may urge the base member 1452 from the elastically deformed state toward the free state and may increase the post-implantation compressive force of the staple 1400 compared to unreinforced polymer staples.

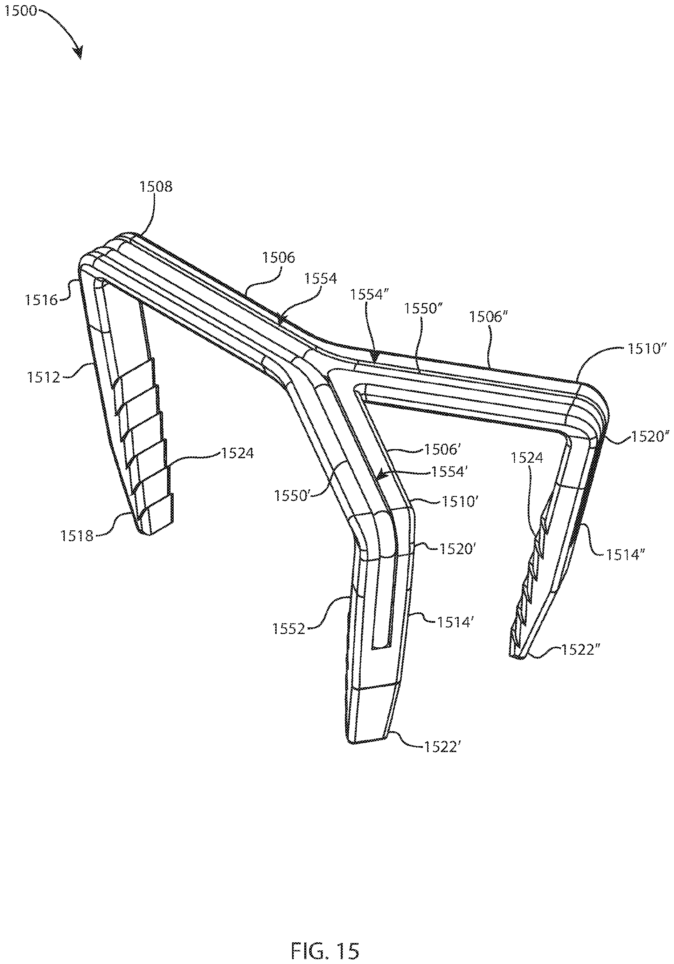

Referring to FIG. 15, yet another staple 1500, not forming part of the claimed invention, includes a bridge 1506 which extends longitudinally between a left end 1508 and a right end. In this example, the right hand portion of the bridge bifurcates into a right front bridge 1506' which extends to a front right end 1510', and a right back bridge 1506" which extends to a back right end 1510". The staple 1500 includes a left leg 1512, a front right leg 1514', and a back right leg 1514". A proximal end 1516 of the leg 1512 is attached to the left end 1508 of the bridge 1506. The leg 1512 terminates in a distal end 1518, which is a free end opposite the bridge 1506. A proximal end 1520' of the leg 1514' is attached to the front right end 1510' of the bridge 1506'. The leg 1514' terminates in a distal end 1522', which is a free end opposite the bridge 1506'. A proximal end 1520" of the leg 1514" is attached to the back right end 1510" of the bridge 1506". The leg 1514" terminates in a distal end 1522", which is a free end opposite the bridge 1506". The facing surfaces of the legs 1512, 1514', 1514" include teeth 1524. When the staple 1500 is implanted, the teeth 1524 help to retain the staple 1500 in the desired position. The teeth 1524 may be on any or all sides of the legs 1512, 1514.

The staple 1500 includes a front reinforcing member 1550' and a back reinforcing member 1550". The reinforcing members 1550', 1550" may be metal, for example a nickel titanium alloy. The reinforcing members 1550', 1550" may have a rectangular, square, or round cross section. The reinforcing members 1550', 1550" may be easily fabricated from ribbon stock, square wire stock, or round wire stock. The reinforcing member 1550' extends along the bridge 1506, 1506' and optionally part way along the legs 1512, 1514'. The reinforcing member 1550' may extend along 50-90% of the length of each leg 1512, 1514'. The reinforcing member 1550" extends along the bridge 1506, 1506" and optionally part way along the legs 1512, 1514". The reinforcing member 1550" may extend along 50-90% of the length of each leg 1512, 1514". The reinforcing members 1550', 1550" may be partially or wholly embedded in the bridge 1506, 1506', 1506" and the legs 1512, 1514', 1514" of the staple 1500, or they may be attached to the surface of the bridge 1506, 1506', 1506" and the legs 1512, 1514', 1514". The staple 1500 may include a base member 1552, which in this example is the polymer portion of the staple 1500 other than the reinforcing members 1550', 1550". The base member 1552 may be easily molded with the desired three-dimensional characteristics for the staple 1500. In this example, the base member 1552 includes channels 1554', 1554" that receive the reinforcing members 1550', 1550" respectively. The channels 1554', 1554" merge into a common channel 1554 towards the left end 1508 of the bridge 1506. The staple 1500 may be referred to as a hybrid staple 1500 because it includes a polymer base member 1552 and metal reinforcing members 1550', 1550".

The staple 1500 is shown in its free state, in which the staple 1500 is not elastically or plastically deflected or deformed. The free state may be referred to as the closed state. In one example of the free state, the staple 1500 experiences no external loads, other than gravity perhaps. However, in the free state, the staple 1500 may experience loads that are below a threshold for elastic or plastic deflection or deformation. In the free state, the distal ends 1518, 1522', 1522" of the legs 1512, 1514', 1514" are closer together than the proximal ends 1516, 1520', 1520" so that the legs 1512, 1514', 1514" converge as they extend away from the bridge 1506, 1506', 1506". The reinforcing members 1550', 1550" may maintain the legs 1512, 1514', 1514" in the free state.

The staple 1500 may be moved between the free state and an elastically deformed state by elastically deforming some or all of the staple 1500, including corresponding portions of the reinforcing members 1550', 1550". For example, elastically deflecting or pressing on one or more of the three bridge portions 1506, 1506', 1506" so that it is straight or curves down between the legs 1512, 1514', 1514" causes one or more of the distal ends 1518, 1522', 1522" to spread apart. Alternatively, one or more of the legs 1512, 1514', 1514" may be elastically deflected so that one or more of the distal ends 1518, 1522', 1522" spread apart or come closer together. The legs 1512, 1514', 1514" may spread apart in the elastically deformed state so as to become parallel. The elastically deformed state may be referred to as the open state. The elastically deformed state may be suitable for implantation of the staple 1500 and may thus be referred to as an insertion state or an implantation state. The reinforcing members 1550', 1550" may urge the base member 1552 from the elastically deformed state toward the free state and may increase the post-implantation compressive force of the staple 1500 compared to unreinforced polymer staples.

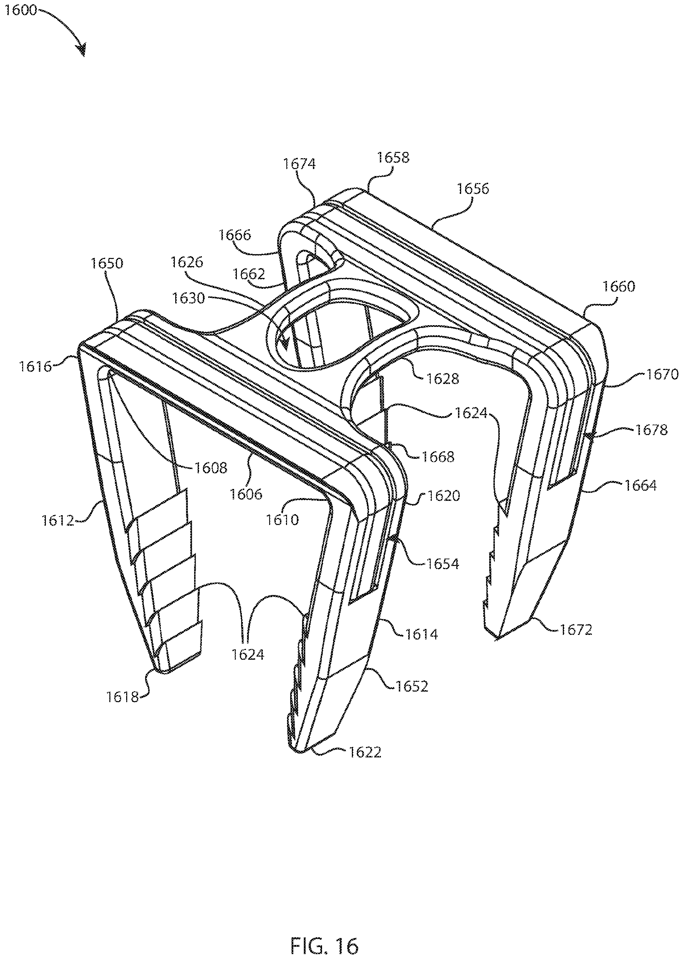

Referring to FIG. 16, yet another staple 1600, not forming part of the claimed invention, includes a front bridge 1606 which extends longitudinally between a left end 1608 and a right end 1610, and a back bridge 1656 which extends longitudinally between a left end 1658 and a right end 1660. The bridges 1606, 1656 are connected together and held spaced apart front to back by left and right arms 1626, 1628. An aperture 1630 extends between the arms 1626, 1628. The staple 1600 includes a front left leg 1612, a front right leg 1614, a back left leg 1662, and a back right leg 1664. A proximal end 1616 of the leg 1612 is attached to the front left end 1608 of the bridge 1606. The leg 1612 terminates in a distal end 1618, which is a free end opposite the bridge 1606. A proximal end 1620 of the leg 1614 is attached to the front right end 1610 of the bridge 1606. The leg 1614 terminates in a distal end 1622, which is a free end opposite the bridge 1606. A proximal end 1666 of the leg 1662 is attached to the back left end 1658 of the bridge 1656. The leg 1662 terminates in a distal end 1668, which is a free end opposite the bridge 1656. A proximal end 1670 of the leg 1664 is attached to the back right end 1660 of the bridge 1656. The leg 1664 terminates in a distal end 1672, which is a free end opposite the bridge 1656.

The facing surfaces of the legs 1612, 1614, 1662, 1664 include teeth 1624. When the staple 1600 is implanted, the teeth 1624 help to retain the staple 1600 in the desired position. The teeth 1624 may be on any or all sides of the legs 1612, 1614, 1662, 1664.

The staple 1600 includes a front reinforcing member 1650 and a back reinforcing member 1674. The reinforcing members 1650, 1674 may be metal, for example a nickel titanium alloy. The reinforcing members 1650, 1674 may have a rectangular, square, or round cross section. The reinforcing members 1650, 1674 may be easily fabricated from ribbon stock, square wire stock, or round wire stock. The reinforcing member 1650 extends along the bridge 1606 and optionally part way along the legs 1612, 1614. The reinforcing member 1650 may extend along 50-90% of the length of each leg 1612, 1614. The reinforcing member 1674 extends along the bridge 1656 and optionally part way along the legs 1662, 1664. The reinforcing member 1674 may extend along 50-90% of the length of each leg 1662, 1664. The reinforcing members 1650, 1674 may be partially or wholly embedded in the bridges 1606, 1656 and the legs 1612, 1614, 1662, 1664 of the staple 1600, or they may be attached to the surface of the bridges 1606, 1656 and the legs 1612, 1614, 1662, 1664. The staple 1600 may include a base member 1652, which in this example is the polymer portion of the staple 1600 other than the reinforcing members 1650, 1674. The base member 1652 may be easily molded with the desired three-dimensional characteristics for the staple 1600. In this example, the base member 1652 includes a front channel 1654 that receives the reinforcing member 1650, and a back channel 1678 that receives the reinforcing member 1674. The staple 1600 may be referred to as a hybrid staple 1600 because it includes a polymer base member 1652 and metal reinforcing members 1650, 1674.

The staple 1600 is shown in its free state, in which the staple 1600 is not elastically or plastically deflected or deformed. The free state may be referred to as the closed state. In one example of the free state, the staple 1600 experiences no external loads, other than gravity perhaps. However, in the free state, the staple 1600 may experience loads that are below a threshold for elastic or plastic deflection or deformation. In the free state, the distal ends 1618, 1622, 1668, 1672 of the legs 1612, 1614, 1662, 1664 are closer together than the proximal ends 1616, 1620, 1666, 1670 so that the legs 1612, 1614, 1662, 1664 converge as they extend away from the bridges 1606, 1656. The reinforcing members 1650, 1674 may maintain the legs 1612, 1614, 1662, 1664 in the free state.

The staple 1600 may be moved between the free state and an elastically deformed state by elastically deforming some or all of the staple 1600, including corresponding portions of the reinforcing members 1650, 1674. For example, elastically deflecting or pressing on the bridge 1606 so that it is straight or curves down between the legs 1612, 1614 causes the distal ends 1618, 1622 to spread apart. The legs 1612, 1614 may spread apart in the elastically deformed state so as to become parallel. Similar manipulations may be made to the bridge 1656 and legs 1662, 1664. The elastically deformed state may be referred to as the open state. The elastically deformed state may be suitable for implantation of the staple 1600 and may thus be referred to as an insertion state or an implantation state. The reinforcing members 1650, 1674 may urge the base member 1652 from the elastically deformed state toward the free state and may increase the post-implantation compressive force of the staple 1600 compared to unreinforced polymer staples.

The staples 1500, 1600 and other staples with three or more legs according to the principles disclosed herein may be included in a suitably modified delivery device, preferably by including a connection for each leg of the staple, wherein the connections may be ruptured after the staple has been implanted as described above.

The delivery devices 100, 300, 500, 600, 700, 800 and staples 102, 302, 502, 602, 702, 802, 900, 1100, 1300, 1400, 1500, 1600 disclosed herein may be provided in packaging, preferably sterile packaging. The packaging may include a delivery device, a staple, and/or an inserter 104, 304, 504, 604, 704, 804. Optionally, the packaging may include a drill guide corresponding to the staple. The drill guide may optionally be provided as a part of the inserter which can be broken off from the inserter for independent use during surgery.

Any methods disclosed herein includes one or more steps or actions for performing the described method. The method steps and/or actions may be interchanged with one another. In other words, unless a specific order of steps or actions is required for proper operation of the embodiment, the order and/or use of specific steps and/or actions may be modified.

Reference throughout this specification to "an embodiment" or "the embodiment" means that a particular feature, structure or characteristic described in connection with that embodiment is included in at least one embodiment. Thus, the quoted phrases, or variations thereof, as recited throughout this specification are not necessarily all referring to the same embodiment.

Similarly, it should be appreciated that in the above description of embodiments, various features are sometimes grouped together in a single embodiment, Figure, or description thereof for the purpose of streamlining the disclosure. This method of disclosure, however, is not to be interpreted as reflecting an intention that any claim require more features than those expressly recited in that claim. Rather, as the following claims reflect, inventive aspects lie in a combination of fewer than all features of any single foregoing disclosed embodiment. Thus, the claims following this Detailed Description are hereby expressly incorporated into this Detailed Description, with each claim standing on its own as a separate embodiment. This disclosure includes all permutations of the independent claims with their dependent claims.

Recitation in the claims of the term "first" with respect to a feature or element does not necessarily imply the existence of a second or additional such feature or element. It will be apparent to those having skill in the art that changes may be made to the details of the above-described embodiments without departing from the underlying principles of the technology.

While specific embodiments and applications of the present technology have been illustrated and described, it is to be understood that the technology is not limited to the precise configuration and components disclosed herein. The scope of the invention is only limited by the appended claims.

## Claims

1. A staple delivery device (100; 300; 500; 600; 700; 800) comprising:
a staple (102; 302; 502; 602; 702; 802) comprising a bridge (106; 306; 506; 606; 706; 806), a first leg (112; 312; 512; 612; 712; 812), and a second leg (114; 314; 514; 614; 714; 814), wherein the bridge (106; 306; 506; 606; 706; 806) extends between a first end (108; 308; 508; 608; 708; 808) and a second end (110; 310; 510; 610; 710; 810), wherein the first leg (112; 312; 512; 612; 712; 812) extends transversely from the first end (108, 108; 308; 508; 608; 708; 808) of the bridge (106; 306; 506; 606; 706; 806) and terminates in a first free end (118; 318; 518; 618; 718; 818) which is remote from the bridge (106; 306; 506; 606; 706; 806), wherein the second leg (114; 314; 514; 614; 714; 814) extends transversely from the second end (110; 310; 510; 610; 710; 810) of the bridge (106; 306; 506; 606; 706; 806) and terminates in a second free end (122; 322; 522; 622; 722; 822) which is remote from the bridge (106; 306; 506; 606; 706; 806), wherein the first free end (118; 318; 518; 618; 718; 818) is beside the second free end (122; 322; 522; 622; 722; 822); and an inserter (104; 304; 504; 604; 704; 804) , where the inserter (104; 304; 504; 604; 704; 804) is connected to the staple (102; 302; 502; 602; 702; 802) or is integrally formed as a single part with the staple (102; 302; 502; 602; 702; 802), wherein the inserter comprises a first body (126; 326; 526; 626;726; 826) and a second body (128; 328; 528; 626; 728; 828) which is a mirror image of the first body (126; 326; 526; 626;726; 826), wherein the first body (126; 326; 526; 626;726; 826) is connected to the first end (108; 308; 508; 608; 708; 808) of the bridge (106; 306; 506; 606; 706; 806) by a first connection (138; 338; 538; 638; 738; 838), wherein the second body (128; 328; 528; 626; 728; 828) is connected to the second end (110; 310; 510; 610; 710; 810) of the bridge (106; 306; 506; 606; 706; 806) by a second connection (140; 340; 540; 640; 740; 840), wherein the first (126; 326; 526; 626;726; 826) and second (128; 328; 528; 626; 728; 828) bodies are joined together at a junction (142; 342; 542; 642; 742; 842) which is adjacent to the bridge (106; 306; 506; 606; 706; 806) opposite the first (112; 312; 512; 612; 712; 812) and second (114; 314; 514; 614; 714; 814) legs;
wherein the inserter (104; 304; 504; 604; 704; 804) is configured to be disconnected from the staple (102; 302; 502; 602; 702; 802) by rupturing the first (138; 338; 538; 638; 738; 838; 938) and second (140; 340; 540; 640; 740; 840; 940) connections, wherein the first (138; 338; 538; 638; 738; 838; 938) and second (140; 340; 540; 640; 740; 840; 940) connections are configured to be ruptured by an action selected from the group consisting of cutting the first (138; 338; 538; 638; 738; 838; 938) and second (140; 340; 540; 640; 740; 840; 940) connections with a surgical wire cutter, cutting the first (138; 338; 538; 638; 738; 838; 938) and second (140; 340; 540; 640; 740; 840; 940) connections with scissors, cutting the first (138; 338; 538; 638; 738; 838; 938) and second (140; 340; 540; 640; 740; 840; 940) connections with a scalpel, breaking the first (138; 338; 538; 638; 738; 838; 938) and second (140; 340; 540; 640; 740; 840; 940) connections with a rupturing instrument, and breaking the first (138; 338; 538; 638; 738; 838; 938) and second (140; 340; 540; 640; 740; 840; 940) connections manually;
**characterised in that** the inserter is configured such that
the junction can be brought into contact with the bridge by moving the first and second bodies to pivot about the first and second connections.

2. A staple delivery device (100; 300; 700; 800) according to claim 1, wherein the first body (126; 326; 726; 826) includes a distal portion (130; 330; 730; 830) and a proximal portion (132; 332; 732; 832) and the second body (128; 328; 728; 828) includes a distal portion (134; 334; 734; 834) and a proximal portion (132; 332; 732; 832), wherein the distal portion (130; 330; 730; 830) of the first body is connected to a proximal end (116; 316; 716; 816) of the first leg (112; 312; 712; 812) by the first connection (138; 338; 738; 838) and the distal portion (134; 334; 734; 834) of the second body is connected to a proximal end (120; 320; 720; 820) of the second leg (114; 314; 714; 814) by the second connection (140; 340; 740; 840), wherein the distal portions (130; 330; 730; 830, 134; 334; 734; 834) are connected to the legs (112; 312; 612; 712; 812, 114; 314; 614; 714; 814), and thus to the bridge (106; 306; 706; 806), solely by the connections (138; 338; 638; 738; 838; 140; 340; 740; 840), which are at or close to the ends (108; 308; 708; 808, 810, 110; 310; 710; 810).

3. A staple delivery device (300; 700) according to claim 1 or 2, wherein the inserter (304; 704) is integrally formed as a single part with the staple (302; 702), the staple (302; 702) comprising a reinforcing member (350; 750) extending along the bridge (306; 706) and optionally part way along the first (312; 712) and second (314; 714) legs.

4. A staple delivery device (300; 700) according to claim 3, wherein the reinforcing member (350; 750) extend along 50-90% of the length of each leg (312, 314; 712,714).

5. A staple delivery device (300; 700) according to claim 3 or 4, wherein the reinforcing member (350; 750) is partially or wholly embedded in the bridge (306; 706) and the first (312; 712) and second (314, 714) legs of the staple (302; 702), or is attached to the surface of the bridge (306; 706) and the first (312; 712) and second (314; 714) legs.

6. A staple delivery device (300; 700) according to any one of claims 3 to 5, wherein the staple (302;702) includes a base member (352; 752), which preferably is a polymer portion of the staple (302; 702) other than the reinforcing member (350; 750).

7. A staple delivery device (300; 700) according to claim 6, wherein the base member (352; 752) includes a channel into which the reinforcing member (350; 750), which is preferably made of metal, is fitted.

8. A staple delivery device (500) according to claim 1, wherein the first body (526) includes a distal portion (530) and a proximal portion (532) and the second body (528) includes a distal portion (534) and a proximal portion (536), wherein the distal portion (530) of the first body is connected to the first end (508) of the bridge (506) by the first connection (538), wherein the first connection (538) is thin and includes a notch (550) at one or both sides of the first connection (538) with the first end (508) of the bridge (506) and the distal portion (534) of the second body is connected to the second end (510) of the bridge (506) by the second connection (540) that is thin and elongated and includes notches (552) on both sides at the connection with the second end (510) of the bridge (506) wherein the distal portions (530, 534) are connected to the bridge (506) solely by the first (538) and second (540) connections, which are at or close to the first (508) and second (510) ends.

9. A staple delivery device (600) according to any one of claims 1, 3 to 5 and 8, comprising: a first securing member (656), and a second securing member (658), wherein the inserter (604) is connected to the staple (602), the first body (626) comprises a first recess (664), wherein the second body (628) comprises a second recess (672), wherein the first recess (664) receives the first securing member (656) to form the first connection (638), wherein the second recess (672) receives the second securing member (658) to form the second connection (640).

10. A staple delivery device (500; 600) according to claim 1, the inserter (504; 604) includes a flex bridge (554; 654) which extends between the first (526) and second (528; 628) bodies, wherein the flex bridge (554;654) is curved or bent so that the flex bridge (554; 654) is deformable, preferably elastically deformable, and maintains the first (526; 626) and second (528; 628) bodies in a desired spatial configuration.

11. A staple delivery device according to claim 9, wherein the first securing member (656) extends from the first end (608) of the bridge (606) opposite the first leg (612), and terminates in a first locking member (660) and the second securing member (658) extends from the second end (610) of the bridge (606) opposite the second leg (614), and terminates in a second locking member (662), wherein each locking member (660, 662) is bilaterally enlarged in a first-second direction relative to its respective securing member (656, 658), so that bilateral undercuts are formed at the transition between the locking member (660, 662) and the securing member (656, 658).

12. A staple delivery device (700; 800) according to claim 2, wherein the distal portions (730; 830, 734; 834) extend along and above the bridge (706; 806) and are joined together at the junction (742; 842) wherein the distal portion (730; 830) of the first body is joined to the junction (742; 842) by a first flexible joint (741; 841) and the distal portion (734; 834) of the second body is joined to the junction (742; 842) by a second flexible joint (743; 843), wherein the junction (742; 842) is not secured to the bridge (706; 806), but can be brought into contact with the bridge (706; 806) and a central component (744; 844) extends proximally from the junction (742; 842) and becomes bilaterally enlarged in a first to second direction as it extends proximally, wherein the proximal side of the central component (744; 844) is flat so that pressure or force may be applied to it manually or with an instrument.

13. A staple delivery device according to claim 1, wherein the first leg (812) comprises a proximal end (816) attached to the first end (808) of the bridge (806) and the first leg (812) terminates in a distal end (818), which is a free end opposite the bridge (806) wherein the second leg (814) comprises a proximal end (820) attached to the second end (810) of the bridge (806) and the second leg (814) terminates in a distal end (822), which is a free end opposite the bridge (806), the proximal ends (816, 820) of the legs (812, 814) are bilaterally enlarged at least in a first to second direction, and optionally in a front to back direction, relative to their respective distal ends (818, 822) wherein the proximal end (816) of the first leg includes a socket (850) which receives the first end (808) of the bridge (806) and the proximal end (820) of the second leg includes a socket (852) which receives the second end (810).

14. Method of manufacturing the delivery device (100;500; 600) according to anyone of claims 1, 8-11, including the steps of:
- forming the staple (102; 502; 602) from a first material; forming the inserter (104; 504; 604) from a second material that may be the same as or different from the first material; and securing the staple (102; 502; 602) and the inserter (104; 504; 604) together; or
- forming the staple (102; 502) from the first material; and forming the inserter (104; 504; 604) from the second material in contact with the previously formed staple (102; 502; 602); or
- forming the inserter (104; 504; 604) from the second material; and forming the staple (102; 502; 602) from the first material in contact with the previously formed inserter (104; 504; 604).

## Patentansprüche

1. Klammerabgabegerät (100; 300; 500; 600; 700; 800), das Folgendes umfasst:
eine Klammer (102; 302; 502; 602; 702; 802) mit einer Brücke (106; 306; 506; 606; 706; 806), einem ersten Schenkel (112; 312; 512; 612; 712; 812) und einem zweiten Schenkel (114; 314; 514; 614; 714; 814), wobei sich die Brücke (106; 306; 506; 606; 706; 806) zwischen einem ersten Ende (108; 308; 508; 608; 708; 808) und einem zweiten Ende (108; 308; 508; 508) erstreckt, wobei sich der erste Schenkel (112; 312; 512; 612; 712; 812) quer vom ersten Ende (108; 308; 508; 608; 708; 808) der Brücke (106; 306; 506; 606; 706; 806) erstreckt und in einem ersten freien Ende (118; 318; 518; 618; 718; 818) endet, das von der Brücke (106; 306; 506; 606; 706; 806) entfernt ist, wobei sich der zweite Schenkel (114; 314; 514; 614; 714; 814) quer vom zweiten Ende (110; 310; 510; 610; 710; 810) der Brücke (106; 306; 506; 606; 706; 806) erstreckt und in einem zweiten freien Ende (122; 322; 522; 622; 722; 822) endet, das von der Brücke (106; 306; 506; 606; 706; 806) entfernt ist, wobei sich das erste freie Ende (118; 318; 518; 618; 718; 818) neben dem zweiten freien Ende (122; 322; 522; 622; 722; 822) befindet; und einen Inserter (104; 304; 504; 604; 704; 804), wobei der Inserter (104; 304; 504; 604; 704; 804) mit der Klammer (102; 302; 502; 602; 702; 802) verbunden oder einstückig als ein einziges Teil mit der Klammer (102; 302; 502; 602; 702; 802) ausgebildet ist, wobei der Inserter einen ersten Körper (126; 326; 526; 626; 726; 826) und einen zweiten Körper (128; 328; 528; 626; 728; 828) umfasst, der ein Spiegelbild des ersten Körpers (126; 326; 526; 626; 726; 826) ist, wobei der erste Körper (126; 326; 526; 626; 726; 826) mit dem ersten Ende (108; 308; 508; 608; 708; 808) der Brücke (106; 306; 506; 606; 706; 806) durch eine erste Verbindung (138; 338; 538; 638; 738; 838) verbunden ist, wobei der zweite Körper (128; 328; 528; 626; 728; 828) mit dem zweiten Ende (110; 310; 510; 610; 710; 810) der Brücke (106; 306; 506; 606; 706; 806) durch eine zweite Verbindung (140; 340; 540; 640; 740; 840) verbunden ist, wobei der erste (126; 326; 526; 626; 726; 826) und der zweite (128; 328; 528; 626; 728; 828) Körper an einer Verbindungsstelle (142; 342; 542; 642; 742; 842) neben der Brücke (106; 306; 506; 606; 706; 806) gegenüber dem ersten (112; 312; 512; 612; 712; 812) und dem zweiten (114; 314; 514; 614; 714; 814) Schenkel aneinandergefügt sind;
wobei der Inserter (104; 304; 504; 604; 704; 804) so konfiguriert ist, dass er von der Klammer (102; 302; 502; 602; 702; 802) durch Brechen der ersten (138; 338; 538; 638; 738; 838; 938) und der zweiten (140; 340; 540; 640; 740; 840; 940) Verbindung getrennt wird, wobei die erste (138; 338; 538; 638; 738; 838; 938) und die zweite (140; 340; 540; 640; 740; 840; 940) Verbindung so konfiguriert sind, dass sie durch eine Aktion gebrochen werden, die ausgewählt ist aus der Gruppe bestehend aus Schneiden der ersten (138; 338; 538; 638; 738; 838; 938) und zweiten (140; 340; 540; 640; 740; 840; 940) Verbindung mit einem chirurgischen Drahtschneider, Schneiden der ersten (138; 338; 538; 638; 738; 838; 938) und zweiten (140; 340; 540; 640; 740; 840; 940) Verbindung mit einer Schere, Schneiden der ersten (138; 338; 538; 638; 738; 838; 938) und zweiten (140; 340; 540; 640; 740; 840; 940) Verbindung mit einem Skalpell, Unterbrechen der ersten (138; 338; 538; 638; 738; 838; 938) und zweiten (140; 340; 540; 640; 740; 840; 940) Verbindung mit einem Rupturierungsinstrument und manuelles Unterbrechen der ersten (138; 338; 538; 638; 738; 838; 938) und zweiten (140; 340; 540; 640; 740; 840; 940) Verbindung; **dadurch gekennzeichnet, dass** der Inserter so konfiguriert ist, dass die Verbindungsstelle mit der Brücke in Kontakt gebracht werden kann, indem der erste und der zweite Körper so bewegt werden, dass sie um die erste und die zweite Verbindung schwenken.

2. Klammerabgabegerät (100; 300; 700; 800) nach Anspruch 1, wobei der erste Körper (126; 326; 726; 826) einen distalen Abschnitt (130; 330; 730; 830) und einen proximalen Abschnitt (132; 332; 732; 832) aufweist und der zweite Körper (128; 328; 728; 828) einen distalen Abschnitt (134; 334; 734; 834) und einen proximalen Abschnitt (132; 332; 732; 832) aufweist, wobei der distale Abschnitt (130; 330; 730; 830) des ersten Körpers mit einem proximalen Ende (116; 316; 716; 816) des ersten Schenkels (112; 312; 712; 812) durch die erste Verbindung (138; 338; 738; 838) verbunden ist und der distale Abschnitt (134; 334; 734; 834) des zweiten Körpers mit einem proximalen Ende (120; 320; 720; 820) des zweiten Schenkels (114; 314; 714; 814) durch die zweite Verbindung (140; 340; 740; 840) verbunden ist, wobei die distalen Abschnitte (130; 330; 730; 830, 134; 334; 734; 834) mit den Schenkeln (112; 312; 612; 712; 812, 114; 314; 614; 714; 814) und somit mit der Brücke (106; 306; 706; 806) allein durch die Verbindungen (138; 338; 638; 738; 838; 140; 340; 740; 840) verbunden sind, die sich an oder nahe den Enden (108; 308; 708; 808, 810, 110; 310; 710; 810) befinden.

3. Klammerabgabegerät (300; 700) nach Anspruch 1 oder 2, wobei der Inserter (304; 704) einstückig als ein einziges Teil mit der Klammer (302; 702) ausgebildet ist, wobei die Klammer (302; 702) ein Verstärkungselement (350; 750) umfasst, das sich entlang der Brücke (306; 706) und optional teilweise entlang des ersten (312; 712) und zweiten (314; 714) Schenkels erstreckt.

4. Klammerabgabegerät (300; 700) nach Anspruch 3, wobei sich das Verstärkungselement (350; 750) entlang 50-90% der Länge jedes Schenkels (312, 314; 712, 714) erstreckt.

5. Klammerabgabegerät (300; 700) nach Anspruch 3 oder 4, wobei das Verstärkungselement (350; 750) ganz oder teilweise in die Brücke (306; 706) und den ersten (312; 712) und zweiten (314, 714) Schenkel der Klammer (302; 702) eingebettet oder an der Oberfläche der Brücke (306; 706) und des ersten (312; 712) und zweiten (314; 714) Schenkels angebracht ist.

6. Klammerabgabegerät (300; 700) nach einem der Ansprüche 3 bis 5, wobei die Klammer (302; 702) ein Basiselement (352; 752) aufweist, das vorzugsweise ein anderer Polymerabschnitt der Klammer (302; 702) als das Verstärkungselement (350; 750) ist.

7. Klammerabgabegerät (300; 700) nach Anspruch 6, wobei das Basiselement (352; 752) einen Kanal aufweist, in den das vorzugsweise aus Metall gefertigte Verstärkungselement (350; 750) eingepasst ist.

8. Klammerabgabegerät (500) nach Anspruch 1, wobei der erste Körper (526) einen distalen Abschnitt (530) und einen proximalen Abschnitt (532) aufweist und der zweite Körper (528) einen distalen Abschnitt (534) und einen proximalen Abschnitt (536) aufweist, wobei der distale Abschnitt (530) des ersten Körpers mit dem ersten Ende (508) der Brücke (506) durch die erste Verbindung (538) verbunden ist, wobei die erste Verbindung (538) dünn ist und eine Kerbe (550) an einer oder beiden Seiten der ersten Verbindung (538) mit dem ersten Ende (508) der Brücke (506) aufweist, und der distale Abschnitt (534) des zweiten Körpers mit dem zweiten Ende (510) der Brücke (506) durch die zweite Verbindung (540) verbunden ist, die dünn und länglich ist und Kerben (552) auf beiden Seiten an der Verbindung mit dem zweiten Ende (510) der Brücke (506) aufweist, wobei die distalen Abschnitte (530, 534) mit der Brücke (506) allein durch die erste (538) und die zweite (540) Verbindung verbunden sind, die sich an oder nahe dem ersten (508) und zweiten (510) Ende befinden.

9. Klammerabgabegerät (600) nach einem der Ansprüche 1, 3 bis 5 und 8, das Folgendes umfasst: ein erstes Befestigungselement (656) und ein zweites Befestigungselement (658), wobei der Inserter (604) mit der Klammer (602) verbunden ist, der erste Körper (626) eine erste Ausnehmung (664) aufweist, wobei der zweite Körper (628) eine zweite Ausnehmung (672) aufweist, wobei die erste Ausnehmung (664) das erste Befestigungselement (656) aufnimmt, um die erste Verbindung (638) zu bilden, wobei die zweite Ausnehmung (672) das zweite Befestigungselement (658) aufnimmt, um die zweite Verbindung (640) zu bilden.

10. Klammerabgabegerät (500; 600) nach Anspruch 1, wobei der Inserter (504; 604) eine Biegebrücke (554; 654) aufweist, die sich zwischen dem ersten (526) und dem zweiten (528; 628) Körper erstreckt, wobei die Biegebrücke (554; 654) gekrümmt oder gebogen ist, so dass die Biegebrücke (554; 654) verformbar, vorzugsweise elastisch verformbar, ist und den ersten (526; 626) und den zweiten (528; 628) Körper in einer gewünschten räumlichen Konfiguration hält.

11. Klammerabgabegerät nach Anspruch 9, wobei sich das erste Befestigungselement (656) vom ersten Ende (608) der Brücke (606) gegenüber dem ersten Schenkel (612) erstreckt und in einem ersten Verriegelungselement (660) endet, und das zweite Befestigungselement (658) sich vom zweiten Ende (610) der Brücke (606) gegenüber dem zweiten Schenkel (614) erstreckt und in einem zweiten Verriegelungselement (662) endet, wobei jedes Verriegelungselement (660, 662) in einer ersten zu zweiten Richtung relativ zu seinem jeweiligen Befestigungselement (656, 658) zweiseitig vergrößert ist, so dass zweiseitige Hinterschneidungen am Übergang zwischen dem Verriegelungselement (660, 662) und dem Befestigungselement (656, 658) gebildet werden.

12. Klammerabgabegerät (700; 800) nach Anspruch 2, wobei sich die distalen Abschnitte (730; 830, 734; 834) entlang und oberhalb der Brücke (706; 806) erstrecken und an der Verbindungsstelle (742; 842) aneinandergefügt sind, wobei der distale Abschnitt (730; 830) des ersten Körpers durch eine erste flexible Verbindungsstelle (741; 841) an die Verbindungsstelle (742; 842) gefügt ist und der distale Abschnitt (734; 834) des zweiten Körpers durch eine zweite flexible Verbindungsstelle (743; 843) an die Verbindungsstelle (742; 842) gefügt ist, wobei die Verbindungsstelle (742; 842) nicht an der Brücke (706; 806) befestigt ist, aber mit der Brücke (706; 806) in Kontakt gebracht werden kann, und eine zentrale Komponente (744; 844) sich proximal von der Verbindungsstelle (742; 842) erstreckt und sich im Laufe ihrer proximalen Erstreckung beidseitig in einer ersten zu zweiten Richtung vergrößert, wobei die proximale Seite der zentralen Komponente (744; 844) flach ist, so dass Druck oder Kraft manuell oder mit einem Instrument auf sie ausgeübt werden kann.

13. Klammerabgabegerät nach Anspruch 1, wobei der erste Schenkel (812) ein proximales Ende (816) aufweist, das am ersten Ende (808) der Brücke (806) angebracht ist, und der erste Schenkel (812) in einem distalen Ende (818) endet, das ein freies Ende gegenüber der Brücke (806) ist, wobei der zweite Schenkel (814) ein proximales Ende (820) aufweist, das am zweiten Ende (810) der Brücke (806) angebracht ist, und der zweite Schenkel (814) in einem distalen Ende (822) endet, das ein freies Ende gegenüber der Brücke (806) ist, die proximalen Enden (816, 820) der Schenkel (812, 814) zweiseitig zumindest in einer ersten zu zweiten Richtung und optional in einer Vorne-nach-hinten-Richtung relativ zu ihren jeweiligen distalen Enden (818, 822) vergrößert sind, wobei das proximale Ende (816) des ersten Schenkels eine Fassung (850) aufweist, die das erste Ende (808) der Brücke (806) aufnimmt, und das proximale Ende (820) des zweiten Schenkels eine Fassung (852) aufweist, die das zweite Ende (810) aufnimmt.

14. Verfahren zur Herstellung des Abgabegeräts (100; 500; 600) nach einem der Ansprüche 1, 8-11, das die folgenden Schritte beinhaltet:
- Bilden der Klammer (102; 502; 602) aus einem ersten Material; Bilden des Inserters (104; 504; 604) aus einem zweiten Material, das das gleiche wie das erste Material oder ein anderes sein kann; und Befestigen der Klammer (102; 502; 602) und des Inserters (104; 504; 604) aneinander; oder
- Bilden der Klammer (102; 502) aus dem ersten Material; und Bilden des Inserters (104; 504; 604) aus dem zweiten Material in Kontakt mit der zuvor ausgebildeten Klammer (102; 502; 602); oder
- Bilden des Inserters (104; 504; 604) aus dem zweiten Material; und Bilden der Klammer (102; 502; 602) aus dem ersten Material in Kontakt mit dem zuvor gebildeten Inserter (104; 504; 604).

## Revendications

1. Dispositif de distribution d'agrafe (100 ; 300 ; 500 ; 600 ; 700 ; 800) comprenant :
une agrafe (102 ; 302 ; 502 ; 602 ; 702 ; 802) comprenant une arcade (106 ; 306 ; 506 ; 606 ; 706 ; 806), une première patte (112 ; 312 ; 512 ; 612 ; 712 ; 812), et une deuxième patte (114 ; 314 ; 514 ; 614 ; 714 ; 814), dans lequel l'arcade (106 ; 306 ; 506 ; 606 ; 706 ; 806) s'étend entre une première extrémité (108 ; 308 ; 508 ; 608 ; 708 ; 808) et une deuxième extrémité (110 ; 310 ; 510 ; 610 ; 710 ; 810), dans lequel la première patte (112 ; 312 ; 512 ; 612 ; 712 ; 812) s'étend à la transversale de la première extrémité (108, 108, 308, 508, 608, 708, 808) de l'arcade (106, 306, 506, 606, 706, 806) et se termine dans une première extrémité libre (118 ; 318 ; 518 ; 618 ; 718 ; 818) qui est éloignée de l'arcade (106 ; 306 ; 506 ; 606 ; 706 ; 806), dans lequel la deuxième patte (114 ; 314 ; 514 ; 614 ; 714 ; 814) s'étend à la transversale de la deuxième extrémité (110 ; 310 ; 510 ; 610 ; 710 ; 810) de l'arcade (106 ; 306 ; 506 ; 606 ; 706 ; 806) et se termine dans une deuxième extrémité libre (122 ; 322 ; 522 ; 622 ; 722 ; 822) qui est éloignée de l'arcade (106 ; 306 ; 506 ; 606 ; 706 ; 806), dans lequel la première extrémité libre (118 ; 318 ; 518 ; 618 ; 718 ; 818) est à côté de la deuxième extrémité libre (122 ; 322 ; 522 ; 622 ; 722 ; 822) ; et une inséreuse (104 ; 304 ; 504 ; 604 ; 704 ; 804), dans lequel l'inséreuse (104 ; 304 ; 504 ; 604 ; 704 ; 804) est raccordée à l'agrafe (102 ; 302 ; 502 ; 602 ; 702 ; 802) ou est formée intégralement comme une seule pièce avec l'agrafe (102 ; 302 ; 502 ; 602 ; 702 ; 802), dans lequel l'inséreuse comprend un premier corps (126 ; 326 ; 526 ; 626 ; 726 ; 826) et un deuxième corps (128 ; 328 ; 528 ; 626 ; 728 ; 828) qui est une image miroir du premier corps (126 ; 326 ; 526 ; 626 ; 726 ; 826), dans lequel le premier corps (126 ; 326 ; 526 ; 626 ; 726 ; 826) est raccordé à la première extrémité (108, 308, 508, 608, 708, 808) de l'arcade (106 ; 306 ; 506 ; 606 ; 706 ; 806) par un premier raccord (138 ; 338 ; 538 ; 638 ; 738 ; 838), dans lequel le deuxième corps (128 ; 328 ; 528 ; 626 ; 728 ; 828) est raccordé à la deuxième extrémité (110 ; 310 ; 510 ; 610 ; 710 ; 810) de l'arcade (106 ; 306 ; 506 ; 606 ; 706 ; 806) par un deuxième raccord (140 ; 340 ; 540 ; 640 ; 740 ; 840), dans lequel le premier (126 ; 326 ; 526 ; 626 ; 726 ; 826) et le deuxième (128 ; 328 ; 528 ; 628 ; 728 ; 828) corps sont joints ensemble à une jonction (142 ; 342 ; 542 ; 642 ; 742 ; 842) qui est adjacente à l'arcade (106 ; 306 ; 506 ; 606 ; 706 ; 806) à l'opposé de la première (112 ; 312; 512; 612; 712 ; 812) et de la deuxième (114 ; 314; 514 ; 614 ; 714 ; 814) patte;
dans lequel l'inséreuse (104 ; 304 ; 504 ; 604 ; 704 ; 804) est configurée pour être détachée de l'agrafe (102 ; 302 ; 502 ; 602 ; 702 ; 802) en rompant le premier (138 ; 338 ; 538 ; 638 ; 738 ; 838, 938) et le deuxième (140 ; 340 ; 540 ; 640 ; 740 ; 840, 940) raccord, dans lequel le premier (138 ; 338 ; 538 ; 638 ; 738 ; 838, 938) et le deuxième (140 ; 340 ; 540 ; 640 ; 740 ; 840, 940) raccord sont configurés pour être rompus par une action sélectionnée parmi le groupe composé de couper le premier (138 ; 338 ; 538 ; 638 ; 738 ; 838, 938) et le deuxième (140 ; 340 ; 540 ; 640 ; 740 ; 840, 940) raccord avec un coupe-fil chirurgical, couper le premier (138 ; 338 ; 538 ; 638 ; 738 ; 838, 938) et le deuxième (140 ; 340 ; 540 ; 640 ; 740 ; 840, 940) raccord avec des ciseaux, couper le premier (138 ; 338 ; 538 ; 638 ; 738 ; 838. 938) et le deuxième (140 ; 340 ; 540 ; 640 ; 740 ; 840, 940) raccord avec un scalpel, casser le premier (138 ; 338 ; 538 ; 638 ; 738 ; 838, 938) et le deuxième (140 ; 340 ; 540 ; 640 ; 740 ; 840, 940) raccord avec un instrument de rupture et casser le premier (138 ; 338 ; 538 ; 638 ; 738 ; 838, 938) et le deuxième (140 ; 340 ; 540 ; 640 ; 740 ; 840, 940) raccord manuellement ;
**caractérisé en ce que** l'inséreuse est configurée de telle sorte que la jonction peut être mise en contact avec l'arcade en déplaçant le premier et le deuxième corps pour qu'ils pivotent autour du premier et du deuxième raccord.

2. Dispositif de distribution d'agrafe (100 ; 300 ; 700 ; 800) selon la revendication 1, dans lequel le premier corps (126 ; 326 ; 726 ; 826) comprend une partie distale (130 ; 330 ; 730 ; 830) et une partie proximale (132 ; 332 ; 732 ; 832) et le deuxième corps (128 ; 328 ; 728 ; 828) comprend une partie distale (134 ; 334 ; 734 ; 834) et une partie proximale (132 ; 332 ; 732 ; 832), dans lequel la partie distale (130 ; 330 ; 730 ; 830) du premier corps est raccordée à une extrémité proximale (116 ; 315 ; 716 ; 816) de la première patte (112 ; 312 ; 712 ; 812) par le premier raccord (138 ; 338 ; 738 ; 838) et la partie distale (134 ; 334 ; 734 ; 834) du deuxième corps est raccordée à une extrémité proximale (120 ; 320 ; 720 ; 820) de la deuxième patte (114 ; 314 ; 714 ; 814) par le deuxième raccord (140 ; 340 ; 740 ; 840), dans lequel les parties distales (130 ; 330 ; 730 ; 830 ; 134 ; 334 ; 734 ; 834) sont raccordées aux pattes (112 ; 312 ; 612 ; 712 ; 812, 114 ; 314 ; 614 ; 714 ; 814), et ainsi à l'arcade (106 ; 306 ; 706 ; 806), seulement par les raccords (138 ; 338 ; 638 ; 738 ; 838 ; 140 ; 340 ; 740 ; 840), qui sont aux ou proches des extrémités (108 ; 308 ; 708 ; 808, 110 ; 310 ; 710 ; 810).

3. Dispositif de distribution d'agrafe (300 ; 700) selon la revendication 1 ou 2, dans lequel l'inséreuse (304 ; 704) est formée intégralement comme une seule pièce avec l'agrafe (302 ; 702), l'agrafe (302 ; 702) comprenant un membre de renforcement (350 ; 750) s'étendant le long de l'arcade (306 ; 706) et optionnellement en partie le long de la première (312 ; 712) et de la deuxième (314 ; 714) patte.

4. Dispositif de distribution d'agrafe (300 ; 700) selon la revendication 3, dans lequel le membre de renforcement (350 ; 750) s'étend le long de 50-90 % de la longueur de chaque patte (312, 314 ; 712 ; 714).

5. Dispositif de distribution d'agrafe (300 ; 700) selon la revendication 3 ou 4, dans lequel le membre de renforcement (350 ; 750) est partiellement ou entièrement intégré dans l'arcade (306 ; 706) et la première (312 ; 712) et la deuxième (314 ; 714) patte de l'agrafe (302 ; 702), ou est attaché à la surface de l'arcade (306 ; 706) et à la première (312 ; 712) et deuxième (314 ; 714) patte.

6. Dispositif de distribution d'agrafe (300 ; 700) selon l'une quelconque des revendications 3 à 5, dans lequel l'agrafe (302 ; 702) comprend un membre de base (352 ; 752), qui est de préférence une partie polymère de l'agrafe (302 ; 702) autre que le membre de renforcement (350 ; 750).

7. Dispositif de distribution d'agrafe (300 ; 700) selon la revendication 6, dans lequel le membre de base (352 ; 752) comprend une rainure dans laquelle le membre de renforcement (350 ; 750), qui est de préférence fabriqué en métal, est adapté.

8. Dispositif de distribution d'agrafe (500) selon la revendication 1, dans lequel le premier corps (526) comprend une partie distale (530) et une partie proximale (532) et le deuxième corps (528) comprend une partie distale (534) et une partie proximale (536), dans lequel la partie distale (530) du premier corps est raccordée à la première extrémité (508) de l'arcade (506) par le premier raccord (538), dans lequel le premier raccord (538) est mince et comprend une encoche (550) d'un coté ou des deux côtés du premier raccord (538) avec la première extrémité (508) de l'arcade (506) et la partie distale (534) du deuxième corps est raccordée à la deuxième extrémité (510) de l'arcade (506) par le deuxième raccord (540) qui est mince et allongé et comprend des encoches (552) des deux côtés au niveau du raccord avec la deuxième extrémité (510) de l'arcade (506), dans lequel les parties distales (530 ; 534) sont raccordées à l'arcade (506) seulement par le premier (538) et le deuxième (540) raccord, qui sont aux ou proches de la première (508) et de la deuxième (510) extrémité.

9. Dispositif de distribution d'agrafe (600) selon l'une quelconque des revendications 1, 3 à 5 et 8, comprenant : un premier membre de fixation (656) et un deuxième membre de fixation (658), dans lequel l'inséreuse (604) est raccordée à l'agrafe (602), le premier corps (626) comprend un premier évidement (664), dans lequel le deuxième corps (628) comprend un deuxième évidement (672), dans lequel le premier évidement (664) reçoit le premier membre de fixation (656) pour former le premier raccord (638), dans lequel le deuxième évidement (672) reçoit le deuxième membre de fixation (658) pour former le deuxième raccord (640).

10. Dispositif de distribution d'agrafe (500 ; 600) selon la revendication 1, l'inséreuse (504 ; 604) comprend une arcade flexible (554 ; 654) qui s'étend entre le premier (526) et le deuxième (528 ; 628) corps, dans lequel l'arcade flexible (554 ; 654) est courbée ou fléchie de sorte que l'arcade flexible (554 ; 654) est déformable, de préférence élastiquement déformable, et maintient le premier (526 ; 626) et le deuxième (528 ; 628) corps dans une configuration spatiale désirée.

11. Dispositif de distribution d'agrafe selon la revendication 9, dans lequel le premier membre de fixation (656) s'étend de la première extrémité (608) de l'arcade (606) à l'opposé de la première patte (612), et se termine dans un premier membre de verrouillage (660) et le deuxième membre de fixation (658) s'étend de la deuxième extrémité (610) de l'arcade (606) à l'opposé de la deuxième patte (614), et se termine dans un deuxième membre de verrouillage (662), dans lequel chaque membre de verrouillage (660, 662) est élargi bilatéralement dans une première à une deuxième direction par rapport à son membre de fixation (656, 658) respectif, de sorte que des dégagements bilatéraux sont formés à la transition entre le membre de verrouillage (660, 662) et le membre de fixation (656, 658).

12. Dispositif de distribution d'agrafe (700 ; 800) selon la revendication 2, dans lequel les parties distales (730 ; 830, 734 ; 834) s'étendent le long et au-dessus de l'arcade (706 ; 806) et sont jointes ensemble à la jonction (742 ; 842) dans lequel la partie distale (730 ; 830) du premier corps est jointe à la jonction (742 ; 842) par un premier joint flexible (741 ; 841) et la partie distale (734 ; 834) du deuxième corps est jointe à la jonction (742 ; 842) par un deuxième joint flexible (743 ; 843), dans lequel la j onction (742 ; 842) n'est pas fixée à l'arcade (706 ; 806), mais peut être mise en contact avec l'arcade (706 ; 806) et un composant central (744 ; 844) s'étend de la j onction de manière proximale (742 ; 842) et devient élargi bilatéralement dans une première à une deuxième direction au fur et à mesure qu'il s'étend de manière proximale, dans lequel le côté proximal du composant central (744 ; 844) est plat de sorte qu'une pression ou force peut être appliquée dessus manuellement ou avec un instrument.

13. Dispositif de distribution d'agrafe selon la revendication 1, dans lequel la première patte (812) comprend une extrémité proximale (816) attachée à la première extrémité (808) de l'arcade (806) et la première patte (812) se termine dans une extrémité distale (818), qui est une extrémité libre à l'opposé de l'arcade (806), dans lequel la deuxième patte (814) comprend une extrémité proximale (820) attachée à la deuxième extrémité (810) de l'arcade (806) et la deuxième patte (814) se termine dans une extrémité distale (822), qui est une extrémité libre à l'opposé de l'arcade (806), les extrémités proximales (816, 820) des pattes (812, 814) sont élargies bilatéralement au moins dans une première à une deuxième direction, et optionnellement dans une direction d'avant en arrière, par rapport à leurs extrémités distales (818, 822) respectives, dans lequel l'extrémité proximale (816) de la première patte comprend une douille (850) qui reçoit la première extrémité (808) de l'arcade (806) et l'extrémité proximale (820) de la deuxième patte comprend une douille (852) qui reçoit la deuxième extrémité (810).

14. Procédé de fabrication du dispositif de distribution (100 ; 500 ; 600) selon l'une quelconque des revendications 1, 8-11, y compris les étapes consistant à :
- former l'agrafe (102 ; 502 ; 602) d'un premier matériau ; former l'inséreuse (104 ; 504 ; 604) d'un deuxième matériau qui peut être le même ou différent du premier matériau ; et fixer l'agrafe (102 ; 502 ; 602) et l'inséreuse (104 ; 504 ; 604) ensemble ; ou
- former l'agrafe (102 ; 502 ; 602) d'un premier matériau ; et former l'inséreuse (104 ; 504 ; 604) d'un deuxième matériau en contact avec l'agrafe (102 ; 502 ; 602) formée précédemment ; ou
- former l'inséreuse (104 ; 504 ; 604) du deuxième matériau ; et former l'agrafe (102 ; 502 ; 602) du premier matériau en contact avec l'inséreuse (104 ; 504 ; 604) formée précédemment.
